# EUROPEAN PATENT SPECIFICATION

(11) **EP 4 243 742 B1**
(45) Date of publication and mention of the grant of the patent: **01.04.2026**
(21) Application number: 21892713.5
(22) Date of filing: 10.11.2021
(51) Int. Cl.: A61F 2/95, A61B 17/22, A61M 25/10, A61M 29/02, A61F 2/24, A61F 2/958

(54) **BALLOON EXPANDABLE TRANSCATHETER VALVE DELIVERY DEVICES AND METHODS**
BALLONEXPANDIERBARE TRANSKATHETERVENTILFREISETZUNGSVORRICHTUNGEN UND VERFAHREN
DISPOSITIFS ET PROCÉDÉS DE POSE DE VALVES TRANSCATHÉTER EXPANSIBLES PAR BALLONNET

(30) Priority: 12.11.2020 US 202063112807 P; 06.08.2021 US 202163230100 P
(43) Date of publication of application: 20.09.2023
(73) Proprietor: Medtronic, Inc., Minneapolis, Minnesota 55432 (US)
(72) Inventor: DUFFY, Niall, Tuam Galway (IE); MULLEN, Conleth, Galway (IE); LYDON, David F., Galway, IE (IE)
(74) Representative: Zimmermann & Partner Patentanwälte mbB
(86) International application number: PCT/US2021/058755
(87) International publication number: WO 2022/103819

(56) References cited:
- WO-A1-2015/141399
- WO-A1-2022/046538
- US-A- 6 027 510
- US-A1- 2004 102 791
- US-A1- 2004 102 791
- US-A1- 2010 185 271
- US-A1- 2013 218 266
- US-B1- 6 264 683

## Description

### Field of the Invention

The present invention is related to systems and methods for transcatheter valve delivery and deployment. In particular, the present invention is related to balloon catheter devices configured for prosthetic heart valve retention during delivery of prosthetic heart valves.

### Background

Transcatheter valve technology provides a minimally invasive means of implanting prosthetic heart valves. The prosthetic heart valve is loaded onto a delivery system that is able to access and navigate the vasculature to the intended implant location and implant the prosthetic heart valve. A conventional approach for a transcatheter valve system is to use a balloon catheter for the delivery system and a prosthetic heart valve incorporating a balloon expandable frame. After reaching the delivery site, the balloon is inflated to expand the prosthetic heart valve into a deployed configuration. After deployment, the balloon is deflated and the balloon catheter is removed.

In conventional balloon catheters, transcatheter balloon expandable prosthetic heart valves are crimped onto the balloon of the balloon catheter. The balloon of the balloon catheter is processed such that the balloon is pleated then folded prior to crimping the prosthetic heart valve onto the balloon. The prosthetic heart valve is delivered to the treatment site and then deployed by inflating the balloon which thereby radially expands the prosthetic heart valve. During delivery procedures, such as gaining vessel access, tracking the balloon catheter through the patient anatomy, crossing the native valve, and advancing the balloon catheter past a distal end of an introducer catheter, forces on the prosthetic heart valve may lead to migration of the prosthetic heart valve on the balloon catheter. Movement of the prosthetic heart valve may lead to an inaccurate deployment position, a prosthetic heart valve that does not fully deploy, and other complications.

Challenges impacting valve retention include the minimum crimp diameter of the prosthetic heart valve, varying contact material and shape between the crimped prosthetic heart valve and the folded balloon, and high forces exerted on the crimped prosthetic heart valve during delivery and deployment procedures. The minimum crimp diameter of the prosthetic heart valve is typically larger than the minimum diameter of the folded balloon. This size mismatch limits contact between the balloon and the prosthetic heart valve and therefore reduces friction forces between the prosthetic heart valve and the balloon. Likewise, the size mismatch limits opportunities for a mechanical lock between the prosthetic heart valve and the balloon (i.e., the prosthetic valve imprinting into the balloon during crimp). Along the length of the crimped prosthetic heart valve, the contact material can change between the metal scaffold and tissue and the balloon giving different friction coefficients and amount of compliance. Further, in the balloon catheter, the crimped prosthetic heart valve typically is the component having the largest diameter. This leads to the prosthetic heart valve being subject to the largest forces during delivery through an introducer.

WO 2022/046538 A1 relates to devices and methods for delivery and deployment of balloon expandable transcatheter valves.

WO 2015/141399 A1 describes an indwelling object delivery system.

US 2004/102791 A1 describes a stent delivery and retention apparatus.

Devices and methods disclosed herein address the issue of prosthetic heart valve migration during delivery.

### SUMMARY

Embodiments hereof relate generally to delivery devices for prosthetic heart valves, and, more specifically, to balloon catheters for prosthetic heart valve delivery and deployment. Balloon catheters consistent with embodiments hereof are configured to reduce or prevent valve migration during delivery and deployment procedures.

The claimed invention is defined in independent claims 1 and 9. Further aspects and preferred embodiments are defined in the dependent claims.

Embodiments hereof include a balloon catheter for deploying a prosthetic heart valve through balloon inflation. The balloon catheter includes an inner shaft defining a guidewire lumen; an outer shaft surrounding the inner shaft defining an inflation lumen between the outer shaft and the inner shaft; a distal portion at a distal end of the outer shaft; a balloon disposed at the distal portion such that fluid delivered to the balloon via the inflation lumen causes the balloon to inflate; a proximal multipart retention bumper having a star shape, the proximal multipart retention bumper being secured to the inner shaft inside the balloon and configurable in an interlocked configuration and a non-interlocked configuration, the proximal multipart retention bumper including a proximal inner wedge and a proximal outer bumper; and a distal multipart star shaped retention bumper having a star shape, the distal multipart retention bumper being secured to the inner shaft inside the balloon and configurable in an interlocked configuration and a non-interlocked configuration, the distal multipart retention bumper including a distal inner wedge and a distal outer bumper.

In further embodiments, a method of assembling a balloon catheter adapted for deploying a prosthetic heart valve through balloon inflation is provided. The method includes disposing a proximal outer bumper and a distal outer bumper over an inner shaft of the balloon catheter at a distal portion of the inner shaft wherein the proximal outer bumper and the distal outer bumper are star shaped; securing a proximal inner wedge and a distal inner wedge to an inner shaft of the balloon catheter at a distal portion of the inner shaft wherein the proximal inner wedge and the distal inner wedge are star shaped; positioning the balloon over the proximal outer bumper, the distal outer bumper, the proximal inner wedge, and the distal inner wedge by passing the proximal outer bumper, the distal outer bumper, the proximal inner wedge, and the distal inner wedge into the balloon via a distal opening of the balloon; interlocking the distal inner wedge and the distal outer bumper to form a distal multipart retention bumper; and interlocking the proximal inner wedge and the proximal outer bumper to form a proximal multipart retention bumper.

### BRIEF DESCRIPTION OF THE DRAWINGS

The accompanying figures, which are incorporated herein, form part of the specification and illustrate embodiments of a prosthesis delivery system. Together with the description, the figures further explain the principles of and enable a person skilled in the relevant art(s) to make and use the balloon catheters described herein. The drawings are provided to illustrate various features of the embodiments described herein and are not necessarily drawn to scale. In the drawings, like reference numbers indicate identical or functionally similar elements.
FIG. 1 illustrates a balloon catheter for prosthetic heart valve delivery and deployment according to embodiments hereof.
FIGS. 2A and 2B illustrate a prosthetic heart valve in expanded and compressed configurations.
FIG. 3 is a graph illustrating forces on a balloon dilation catheter in various size introducers.
FIGS. 4A and 4B illustrate a balloon catheter employing retention bumpers as valve retention devices according to embodiments hereof.
FIGS. 5A-5F illustrate multipart retention bumpers according to embodiments hereof.
FIGS. 6A-6H illustrate stages of an assembly process for a balloon catheter employing multipart retention bumpers.
FIGS. 7A-7G illustrate multipart retention bumpers according to embodiments hereof.
FIGS. 8A-8G illustrate stages of an assembly process for a balloon catheter employing multipart retention bumpers.
FIGS. 9A-9H illustrate multipart retention bumpers according to embodiments hereof.
FIGS. 10A-10B illustrate a balloon catheter using retention bumpers according to embodiments hereof.
FIGS. 11A-B illustrate tools configured to facilitate the interlocking of multipart bumpers according to embodiments described herein.
FIG. 12 is a flow chart of a balloon catheter assembly process for assembly of a balloon enabled prosthetic heart valve delivery and deployment system consistent with embodiments described herein.

### DETAILED DESCRIPTION OF THE INVENTION

Specific embodiments of the present invention are now described with reference to the figures. Unless otherwise indicated, for the balloon catheters discussed herein, the terms "distal" and "proximal" are used in the following description with respect to a position or direction relative to the treating clinician or operator. "Distal" and "distally" are positions distant from or in a direction away from the clinician, and "proximal" and "proximally" are positions near or in a direction toward the clinician. As used herein, the term "proximal force" refers to a force in the proximal direction and the term "distal force" refers to a force in the distal direction.

The following detailed description is merely illustrative in nature and is not intended to limit the invention or the application and uses of the invention. Although the description of the invention is in the context of balloon catheter enabled delivery and deployment of prosthetic heart valves, aspects of the invention may also be used in any other context that is useful. As an example, the description of the invention is in the context of delivery and deployment of heart valve prostheses. As used herein, "prosthesis" or "prostheses" may include any prosthesis including a balloon expandable structure. Modifications can be made to the embodiments described herein without departing from the scope of the present invention. Therefore, the following detailed description is not meant to be limiting. Furthermore, there is no intention to be bound by any expressed or implied theory presented in the preceding technical field, background summary or the following detailed description. <Insert new page 4a>

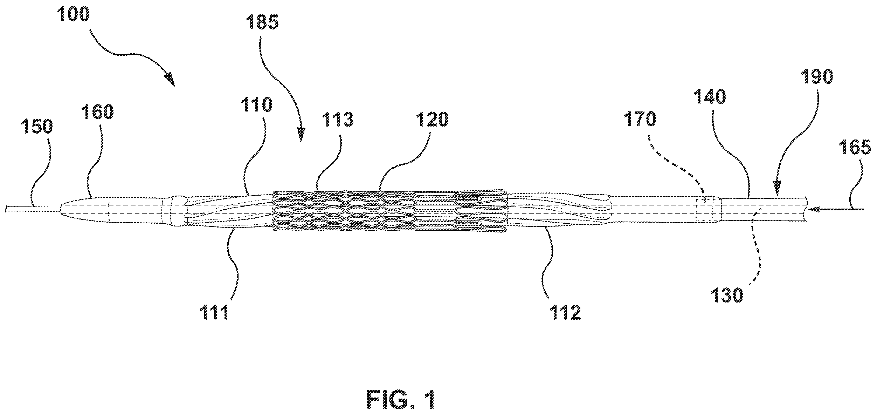

FIG. 1 illustrates a balloon catheter 100 for prosthetic heart valve delivery and deployment. The balloon catheter 100 includes an inflatable balloon 110, an outer shaft 140, and an inner shaft 130. A prosthetic heart valve 120 may be crimped onto the inflatable balloon 110. The description uses the unit "inch", wherein 1 inch corresponds to 2.54 cm.
The inner shaft 130 defines a guidewire lumen 165 such that the balloon catheter 100 may be slidably disposed and tracked over a guidewire 150. The outer shaft 140 defines an annular balloon inflation lumen 170 disposed between the inner shaft 130 and the outer shaft 140. The balloon 110 is disposed on the balloon catheter 100 at a delivery portion 185 and includes a distal portion 111, a proximal portion 112, and a central portion 113. The delivery portion 185 is located at a distal end of the outer shaft 140. Proximal to the delivery portion 185 is a proximal portion 190 of the balloon catheter 100, which includes the outer shaft 140 and proximal portions of the inner shaft 130. The proximal portion 112 of the balloon 110 is disposed over the outer shaft 140. The outer shaft 140 may be tapered or stepped to a smaller diameter to accommodate the balloon 110. The distal portion 111 of the balloon may be retained by the distal tip 160 of the balloon catheter 100 or it may be attached to a distal portion of the inner shaft 130 proximal of the distal tip 160. Both the inner shaft 130 and the outer shaft 140 extend proximally and terminate in a handle or housing (not shown) of the balloon catheter 100.

The outer shaft 140 extends into the interior of the balloon 110 and terminates therein at an open distal end, while the inner shaft 130, defining the guidewire lumen 165, extends to the distal tip 160 and terminates therein. This arrangement permits the balloon inflation lumen 170 to carry fluid pumped from the handle of the balloon catheter 100 into the interior of the balloon 110. The release of fluid to the interior of the balloon 110 causes the balloon 110 to inflate, an action that is employed to expand a prosthetic heart valve frame and deploy the prosthetic heart valve 120.

The prosthetic heart valve 120 has a minimum size in a collapsed state beyond which it cannot be collapsed or compressed any further. The frame of the prosthetic heart valve 120 permits only a certain amount of compression. In a balloon catheter 100, this minimum size may not be small enough to create a large amount of contact between the balloon 110 and the prosthetic heart valve 120. In other embodiments, the frame of the prosthetic heart valve 120 may be configured for tighter crimping. Tighter crimping, however, may cause the crimped balloon 110 to interfere with fluid flowing into the balloon for inflation, leading to irregularities in deployment. These limitations on compression of the prosthetic heart valve 120 may thus prevent the prosthetic heart valve 120 from being crimped tightly onto the balloon 110. A looser crimping of the prosthetic heart valve 120 can result in a less secure fitting and a potential for migration of the prosthetic heart valve 120 when subject to proximal or distal forces during delivery.

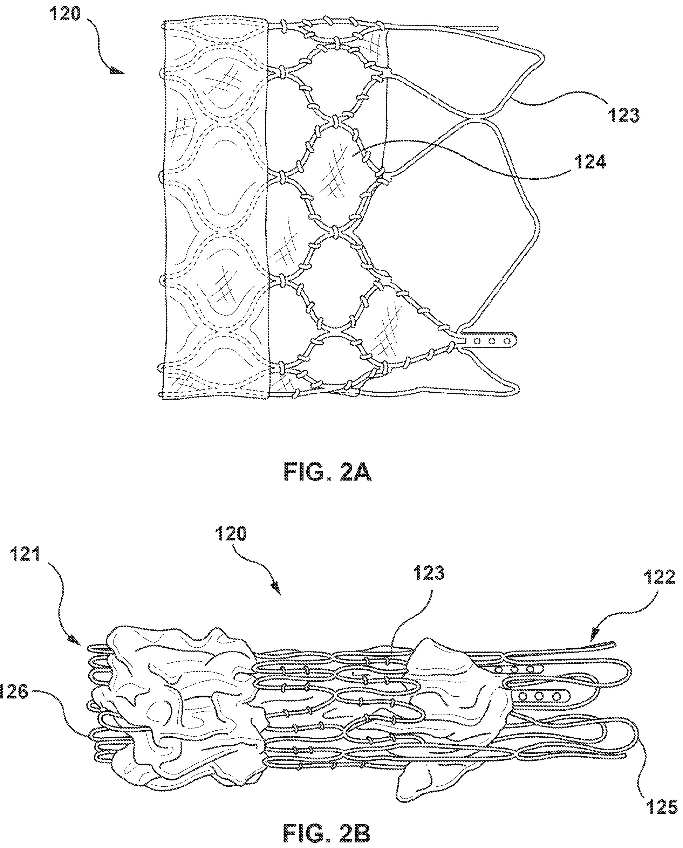

FIGS. 2A and 2B illustrate an example the prosthetic heart valve 120 in expanded and collapsed configurations. The prosthetic heart valve 120 includes a balloon expandable frame 123 and prosthetic valve tissue 124. In a pre-deployment state, the prosthetic heart valve 120 has a proximal end 122 and a distal end 121, which refer to the orientation of the prosthetic heart valve 120 as loaded onto the balloon catheter 100. In the embodiment shown, the distal end 121 is an inflow end of the prosthetic heart valve 120 and the proximal end 122 is an outflow end of the prosthetic heart valve 120. The frame 123 includes a plurality of crowns 125 at the proximal end 122 and a plurality of crowns 126 at the distal end 121. The crowns 125 are bends or tums in the frame structure. The prosthetic heart valve 120 may be non-symmetrical, having different expandable frame 123 geometry and prosthetic valve tissue 124 construction at the proximal end 122 and the distal end 121. Other prosthetic heart valves may differ in design, having expandable frames that differ in structure and construction and prosthetic heart valve elements that also differ. Accordingly, different prosthetic heart valves may react differently under balloon expansion deployment, due to variations in structural characteristics, such as radial stiffness. Because different prosthetic heart valves react differently to expansion and compression, embodiments hereof are designed to prevent migration of prosthetic heart valves regardless of their structure and construction.

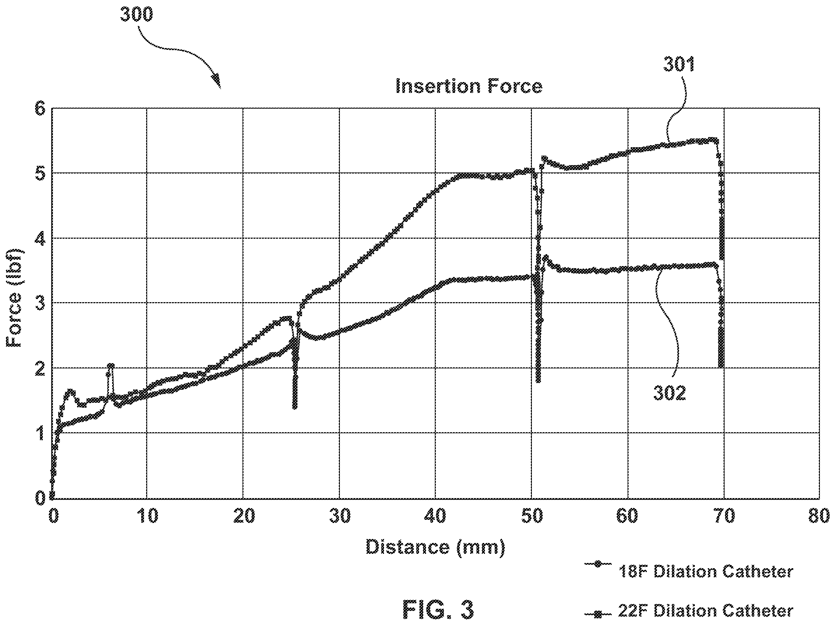

FIG. 3 illustrates a graph of forces associated with the passage of devices through introducers of different sizes. Chart 300 illustrates forces measured during insertion of a 22 french balloon dilation catheter and an 18 french balloon dilation catheter into a 22 french introducer manufactured by Cook Medical^{®}. Plot 301 shows that the introduction of a 22 french balloon dilation catheter into a 22 french Cook Medical^{®} introducer required up to 5.5 lbf while plot 302 shows that the introduction of an 18 french balloon dilation catheter into a 22 french Cook Medical^{®} introducer required up to 3.5 lbf. Balloon catheters for delivery of prosthetic heart valves typically have a profile between 18F and 22F. Accordingly, the chart 300, although it shows the forces on balloon dilation catheters, provides an indication of the types of forces that a balloon catheter for prosthetic heart valve delivery is likely to be subject to. When using a balloon catheter without a sheath, the prosthetic heart valve contacts the interior wall of the introducer. Therefore, the prosthetic heart valve is subject to a significant amount of the force shown in chart 300 during delivery. This force may be large enough to cause the prosthetic heart valve to migrate on the balloon catheter during delivery.

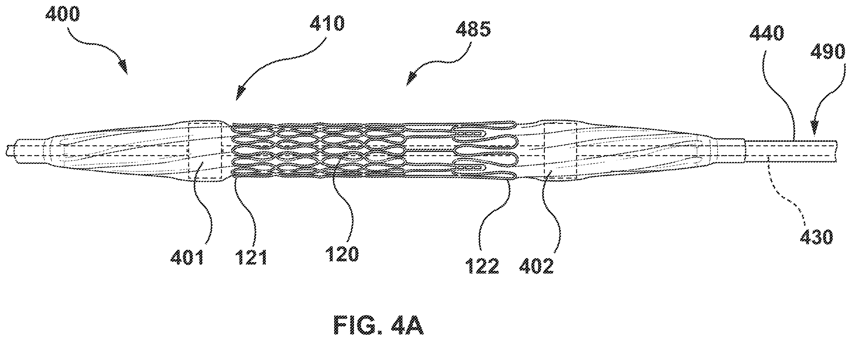

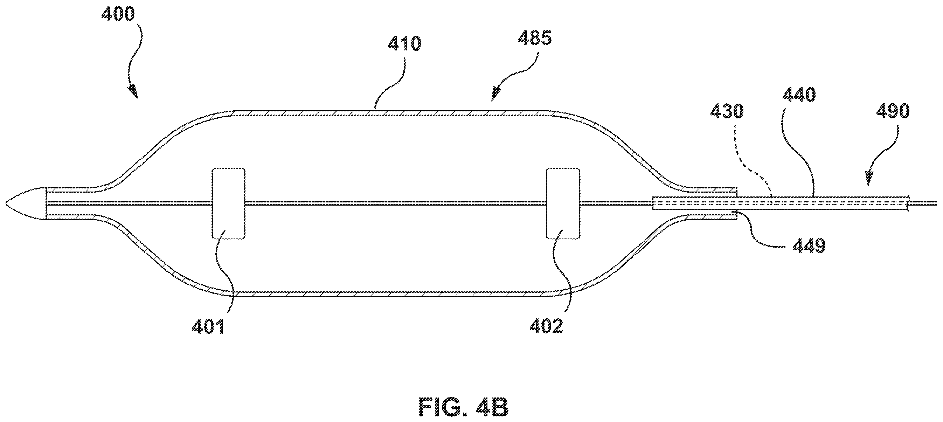

Balloon catheters consistent with embodiments hereof are configured to deliver and deploy, through the use of an inflatable balloon, transcatheter balloon expandable prosthetic heart valves (referred to herein as "prosthetic heart valves"). Balloon catheters consistent with embodiments hereof include one or more valve retention devices configured to prevent or reduce valve migration during delivery and deployment. Balloon catheters including valve retention devices and methods of their use consistent with embodiments hereof are described below with respect to FIGS. 4-10.

FIG. 4A illustrates a balloon catheter 400 employing retention bumpers as valve retention devices according to embodiments hereof. Retention bumpers 401, 402 act as valve retention devices to maintain the position of a prosthetic heart valve such as the prosthetic heart valve 120 mounted on the balloon catheter 400. Balloon catheter 400 further includes a balloon 410, an inner shaft 430, and an outer shaft 440. The balloon catheter 400 further includes a delivery portion 485 and a proximal portion 490. Also illustrated in FIG. 4A is the prosthetic heart valve 120 crimped onto the balloon 410. The retention bumpers 401, 402 are disposed in the delivery portion 485 of the balloon catheter 400 beneath or within the balloon 410. The retention bumpers 401, 402 are attached to the inner shaft 430. In further embodiments, retention bumpers 401, 402 may be secured via alternative means.

The retention bumpers 401, 402 function to maintain an axial position of the prosthetic heart valve 120. The retention bumpers 401, 402 are positioned adjacent to the prosthetic heart valve 120 so as to maintain an axial position, i.e., prevent or reduce axial migration, through contact. In particular, as shown in FIG. 4A, the retention bumper 402 is positioned adjacent the proximal end 122 of the prosthetic heart valve 120 and the retention bumper 401 is positioned adjacent the distal end 121 of the prosthetic heart valve 120. When subject to proximal or distal forces, the prosthetic heart valve 120 presses up against the portion of the balloon 410 raised by the retention bumpers 401, 402, which arrests the movement of the prosthetic heart valve 120.

The retention bumpers 401, 402 may also function to reduce forces acting on the prosthetic heart valve 120. The retention bumpers may be sized such that they (or the balloon 410 covering them) extend further radially outward than the prosthetic heart valve 120. Thus, the features of the balloon catheter 400 having the largest diameter are the retention bumpers 401, 402 and not the prosthetic heart valve 120. When inserted into an introducer, the retention bumpers 401, 402 therefore are the portion of the balloon catheter 400 that contacts the introducer wall, thereby preventing the wall of the introducer from contacting the prosthetic heart valve 120 and thereby reducing the amount of force acting on the prosthetic heart valve 120 from the inner walls of the introducer.

FIG. 4B illustrates an assembly of the balloon catheter 400 employing retention bumpers as valve retention devices according to embodiments hereof. The retention bumpers 401/402 are disposed on the inner shaft 430, inside of the balloon 410. The balloon 410 has a balloon opening 449 at its proximal end, its distal end, or both. The balloon opening 449 has a diameter of approximately 0.2 inches. In further embodiments, the balloon opening 449 may have a diameter of anywhere between approximately 0.1 inches and 0.25 inches. The retention bumpers 401/402 each have an outer diameter of approximately 0.3 inches. In further embodiments, the retention bumpers 401/402 may each have a diameter of anywhere between approximately 0.25 inches and 0.35 inches. The retention bumpers 401/402 have outer diameters larger than the diameter of the balloon opening 449. Due to this imbalance in size, assembly of a balloon catheter 400 with solid and/or rigid retention bumpers 401/402 may be challenging.

The balloon opening 449 and all balloon openings referred to herein are not required to be circular. Balloon openings and/or balloon necks discussed herein may be circular, elliptical, and/or any other suitable shape. In embodiments, catheter balloons may be flexible and thus may adopt any shape which they may be formed into.

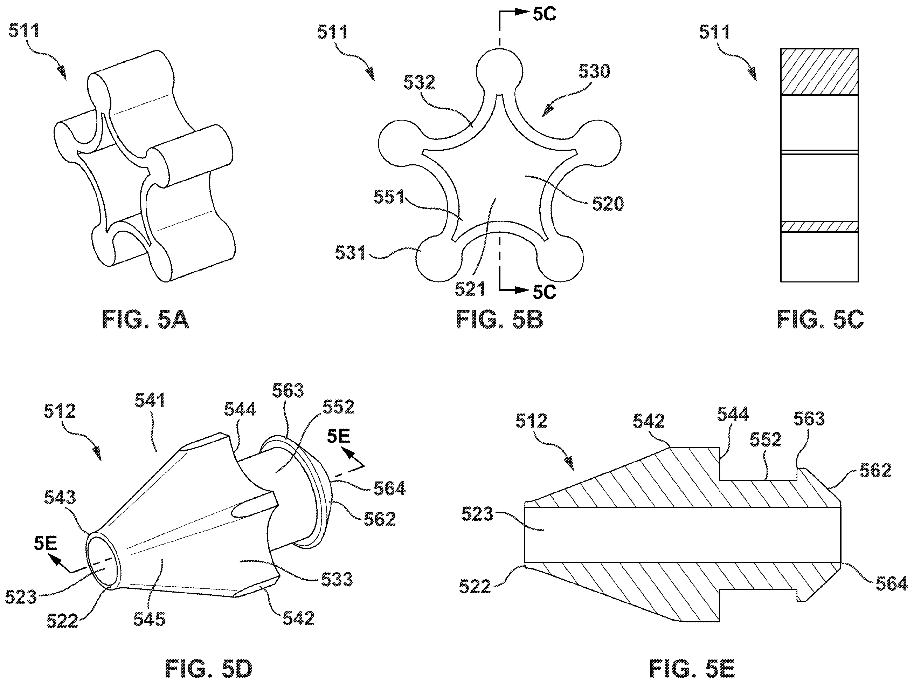

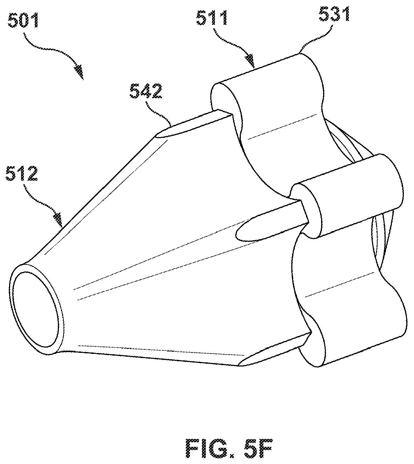

In embodiments, as described below with respect to FIGS. 5-9, retention bumpers and balloons are constructed to mitigate challenges associated with the size discrepancy between outer bumper diameter and balloon opening diameter. Embodiments described herein include retention bumpers having collapsible and expandable characteristics as well as catheters having novel assembly and processing steps. All retention bumpers described herein may be employed to prevent prosthetic heart valve migration during delivery and deployment and to facilitate balloon catheter assembly, as described above. The embodiments illustrated in FIGS. 5-9 may include all features of the balloon catheter 400, whether stated or not. All features of the embodiments described herein may be suitably combined with features of other embodiments.

Retention bumpers described herein may be illustrated or described with respect to a specific profile. Unless otherwise stated, a retention bumper profile refers to the profile when viewed perpendicularly to a central axis. For example, FIGS. 4A and 4B show generally cylindrical retention bumpers that are generally rectangular in profile when viewed. Unless explicitly stated otherwise, bumper profiles illustrated or described with respect to a specific embodiment are non-limiting examples only, and any suitable bumper profile may be employed with any embodiments described herein. Suitable bumper profiles include tapered profiles, spherical profiles, cylindrical profiles, and others.

In embodiments, any or all of the retention bumpers described herein may include radiopaque markers to assist during prosthetic heart valve delivery. In embodiments, any or all of the retention bumpers described herein may be manufactured and assembled by any suitable manufacturing process or technique.

FIGS. 5A-5F illustrate multipart retention bumpers 501 consistent with embodiments hereof. FIGS. 5A-5C illustrate an outer bumper 511 and FIGS. 5D and 5E illustrate an inner wedge 512. FIG. 5F illustrates the multipart retention bumper 501 including the inner wedge 512 and the outer bumper 511 in an interlocked configuration. The outer bumper 511 and the inner wedge 512 are configured such that the outer bumper 511 may be stretched over the inner wedge 511, causing the radius of the outer bumper 511 to expand. Each of the outer bumper 511 and the inner wedge 512 may have a star shape, with an inner core or hub and extensions, arms, or spokes extending radially.

FIG. 5A is a perspective view of the outer bumper 511, FIG. 5B is a plan view of the outer bumper 511, and FIG. 5C is a cutaway profile view taken at the line 5C-5C of FIG. 5B. The multipart retention bumpers 501 include inner wedges 512 and outer bumpers 511. The inner wedge 512 and outer bumper 511 are configured such that, when interlocked, the maximum diameter of the outer bumper 511 (and thus the maximum diameter of the multipart retention bumpers 501) expands from a first radially unexpanded size to a second radially expanded size, wherein the first radially unexpanded size is selected to fit within the neck of a balloon during assembly of a balloon catheter and the second radially expanded size is selected to permit the interlocked multipart retention bumper 501 to maintain the axial position of a prosthetic heart valve crimped to the balloon, as discussed in greater detail below.

As used herein, the terms "radially unexpanded" and "radially expanded" refer to the expansion of the outer bumpers described herein when combined with the inner wedges described herein. In the radially expanded state, an outer bumper has an increased effective diameter relative to the radially unexpanded state, where "effective diameter" refers to the diameter required for a circle to circumscribe the outer bumper. The radially unexpanded state of the outer bumper is the initial or resting state of the outer bumper when there are no external forces placed on it. The radially unexpanded state of the outer bumper may also refer to the as-manufactured state or as-molded state.

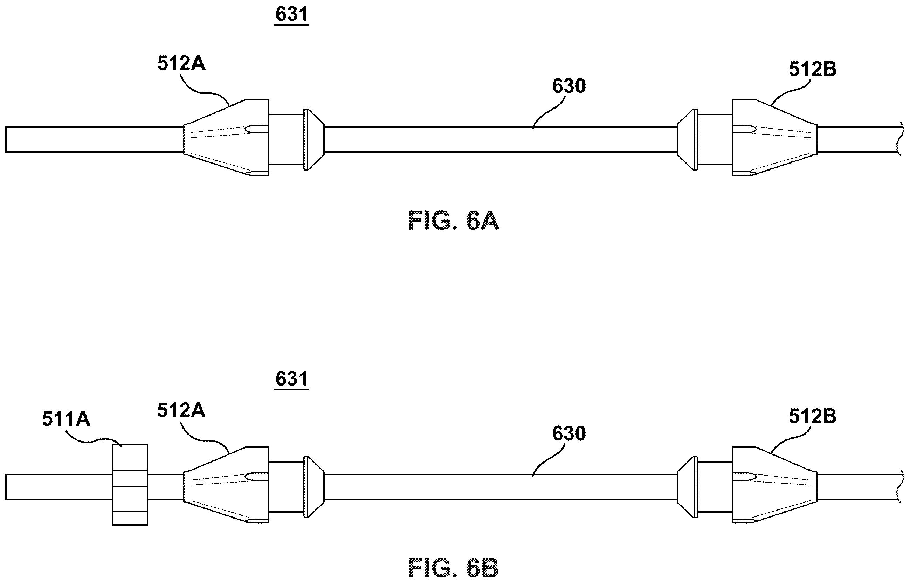

In embodiments, each of the inner wedges 512 and outer bumpers 511 are configured with maximum diameters between approximately 0.1 inches and 0.25 inches, between 0.15 and 0.22 inches, between 0.19 and 0.21 inches, or approximately 0.2 inches. Such sizes may be suitable for entry into the opening of a balloon of a balloon catheter, as discussed in greater detail below with respect to FIGS. 6A-6H. In embodiments, the multipart retention bumpers 501 may have a maximum diameter between 0.25 inches and 0.35 inches, between 0.27 and 0.33 inches, between 0.29 and 0.31 inches, or approximately 0.3 inches after the inner wedges 512 and the outer bumpers 511 are interlocked.

The outer bumper 511 is formed from any suitable flexible material, including elastic materials, such as polymers, elastomers, thermoplastic elastomers, PTFE, PS, PET, etc. and is configured to expand. In embodiments, the outer bumper 511 may be formed from a thermoplastic elastomer (TPE) such as Pebax^{®} 55. The outer bumper 511 includes a spoked perimeter 530 surrounding a hollow core 520. The spoked perimeter 530 includes a plurality of spokes 531 that project outward from an inner perimeter 532. The spoked perimeter 530 surrounds the hollow core 520, which is an empty interior space including a central core 521 and core extensions 551. The core extensions 551 extend radially from the central core 521 towards each of the spokes 531. The hollow core 520 comprising the central core 521 and the core extensions 551 is generally star shaped. The walls of the spoked perimeter are substantially parallel to each other and are configured to mate with the cylindrical features of the seating hub 552, as discussed further below.

The inner wedge 512 includes a central hub 522 surrounding an interior cavity 523. As shown, e.g., in FIGS. 6A-6H, the interior cavity 523 is sized and configured suitably to accommodate an inner shaft of a balloon catheter. The inner wedge 512 further includes three sections disposed over the central hub 522. The first section, disposed at one end of the central hub 522, is a ridged tapered body 541 having a star shape. The second section, disposed in a middle portion of the central hub 522, is a seating hub 552. The third section disposed at the end of the central hub 522 opposite the ridged tapered body 541 is a tapered end 562.

The ridged tapered body 541 includes a tapered base 545, extension arms 542, an apex 543 at the end of the outer bumper 511, and a base 544 at the intersection between the ridged tapered body 541 and the seating hub 552. The star shape of the ridged tapered body 541 is defined by the tapered base 545 and the extension arms 542. The diameter of the apex 543 is smaller than the diameter of the base 544. Extension arms 542 extend radially from the tapered base 545. The extension arms 542 have a taper, such that they extend further from the tapered base 545 at the base 544 than at the apex 543. Accordingly, the extension arms 542 define a largest effective diameter of the inner wedge 512 at the base 544 of the ridged tapered body 541. The interior cavity 523 runs axially through the ridged tapered body 541. The spaces between the extension arms 542 are flow recesses 533. The flow recesses 533 extend from the tapered base 545 to a diameter defined by the extension arms 542.

The inner wedge 512 further includes a seating hub 552 adjacent to the base 544 of the ridged tapered body 541. The seating hub 552 is a hollow cylinder, with the hollow occupied by the interior cavity 523. The outer diameter of the seating hub 552 is greater than the diameter of the interior cavity 523 and less than the diameter of the base 544 of the ridged tapered body 541. Thus, the base 544 of the ridged tapered body 541 forms a flange or stop at one side of the seating hub 552.

The inner wedge 512 further includes the tapered end 562. The tapered end 562 is a tapered shape with a base 563 adjacent the seating hub 552 and an apex 564 at the end of the inner wedge 512. The base 563 of the tapered end 562 has an outer diameter greater than the outer diameter of the seating hub 552. Thus, the base 563 of the tapered end 562 forms a flange or stop at a side of the seating hub 552 opposite the ridged tapered body 541.

The inner wedge 512 is formed from a suitable rigid material, including suitable plastics or polymers, including high density polyethylene (HDPE), TPE, Pebax^{®} and any other suitable rigid material. In embodiments, the material of the inner wedge 512 is selected so as to be substantially non-compressible during conditions expected to accompany manufacture, assembly, or use. As used herein, substantially non-compressible during conditions expected to accompany manufacture, assembly, or use means that the inner wedges 512 show less than 5%, less than 3%, and/or less than 1% strain when subject to forces or stresses common during manufacture, assembly, and/or use. For example, a suitable durometer may be in excess of 60D, in excess of 65D, in excess of 70D, or in excess of 75D.

FIG. 5F illustrates the multipart retention bumper 501 interlocked, with the outer bumper 511 disposed over the seating hub 552 of the inner wedge 512. The outer bumper 511 is configured to stretch, i.e., elastically deform, around the inner wedge 512 and to be disposed on the seating hub 552 of the inner wedge 512. The seating hub 552 of the inner wedge 512 has a diameter greater than that of the hollow core 520 such that, when the outer bumper 511 is disposed on the seating hub 552, it remains in a stretched configuration. The stretched configuration of the outer bumper 511 causes an increase in its effective diameter. As used herein, "effective diameter," refers to the diameter of a circle circumscribing the spokes 531 of the outer bumper 511.

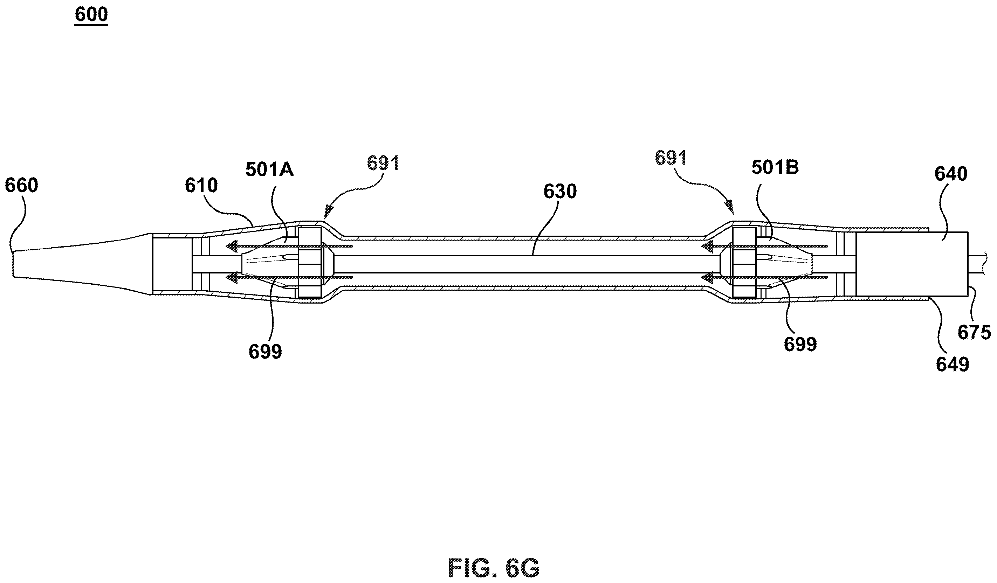

When disposed over the seating hub 552, the spokes 531 of the outer bumper 511 have an increased effective diameter due to stretching caused by the diameter of the seating hub 552. The outer bumper 511 may be disposed on the seating hub 552 via stretching over either the tapered end 562 or over the ridged tapered body 541. Once seated, the spokes 531 of the outer bumper 511 are arranged in radial alignment with the extension arms 542 of the inner wedge 512. This arrangement allows inflation fluid to flow between the extension arms 542 (in the flow recesses 533) and between the spokes 531, without significant obstruction. Further, the extension arms 542 of the inner wedge 512 provide lateral support for the spokes 531 of the outer bumper 511. These features are discussed in greater detail below with respect to FIG. 6G.

Prior to stretching over the inner wedge 512, the outer bumper 511 has an effective diameter at the first radially unexpanded size smaller than the balloon opening. After stretching, the outer bumper 511 has an effective diameter at the second radially expanded size great enough to retain a prosthetic heart valve. Seating the outer bumper 511 on the seating hub 552 of the inner wedge 512 causes the effective diameter of the outer bumper 511 to expand from the first radially unexpanded size to the second radially expanded size.

FIGS. 6A-6H illustrate steps during assembly of the distal end of the balloon catheter 600. FIGS. 6A-6H illustrate an assembly process employing the multipart retention bumpers 501 for example purposes only. The assembly process of FIGS. 6A-6H may be suitable for multipart retention bumpers 501. The assembly process of FIGS. 6A-6H is by way of example only, and balloon catheters including multipart retention bumpers may be assembled using different steps in a different order without departing from the scope of the present disclosure.

FIG. 6A illustrates an inner shaft 630 prepared for balloon catheter assembly. The partial assembly 631 includes the inner shaft 630 and distal and proximal inner wedges 512A / 512B secured thereto. Over molding of the inner wedges 512A / 512B onto the inner shaft 630 may be selected to provide a sure connection throughout usage of the balloon catheter, facilitate manufacturing, and insure accurate placement of the inner wedges 512A / 512B on the inner shaft 630. In further embodiments, the inner wedges 512A / 512B may be secured to the inner shaft 630 via other techniques, such as by bonding and/or adhesive.

FIG. 6B illustrates a step in the assembly of the distal end of the balloon catheter 600. The distal outer bumper 511A is loaded over the inner shaft 630, distal of both of the inner wedges 512A / 512B, but not secured thereto.

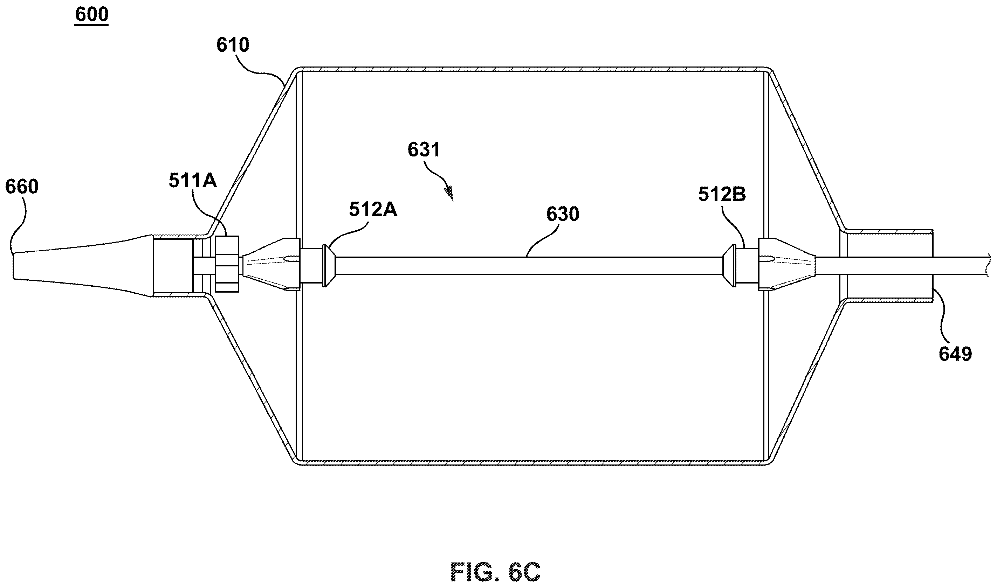

FIG. 6C illustrates a step in the assembly of the distal end of the balloon catheter 600. The balloon 610 and distal tip 660 are added to the partial assembly 631. The partial assembly 631, including the inner shaft 630, inner wedges 512A / 512B, and the distal outer bumper 511A, is slid into the balloon 610 through the proximal balloon opening 649 of the balloon 610. The inner shaft 630 is secured to the distal tip 660 by any suitable means, e.g., adhesive, heat bonding, etc. The balloon 610 is then secured to the distal tip via any suitable means. As discussed above, the inner wedges 512A / 512B and the outer bumpers 511A / 511B are sized with an effective diameter at the first radially unexpanded size, sufficiently narrow for passing through the proximal balloon opening 649 of the balloon 610 without compression of any type.

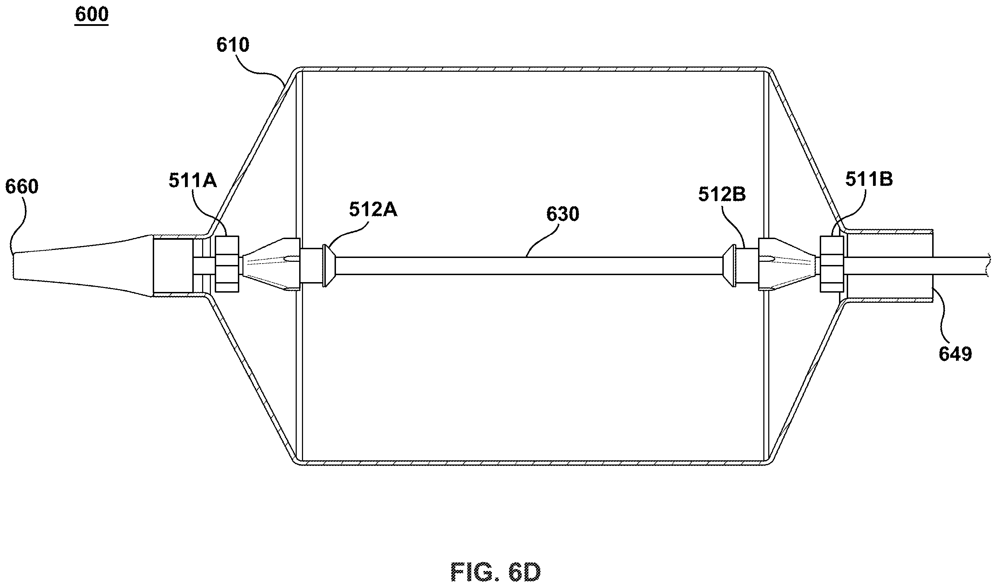

FIG. 6D illustrates a step in the assembly of the distal end of the balloon catheter 600. The second, proximal inner wedge 512B is disposed over the inner shaft 630 and moved into the interior of the balloon 610. The proximal inner wedge 512B is sized with an effective diameter such that it passes through the proximal balloon opening 649 of the balloon 610 without compression.

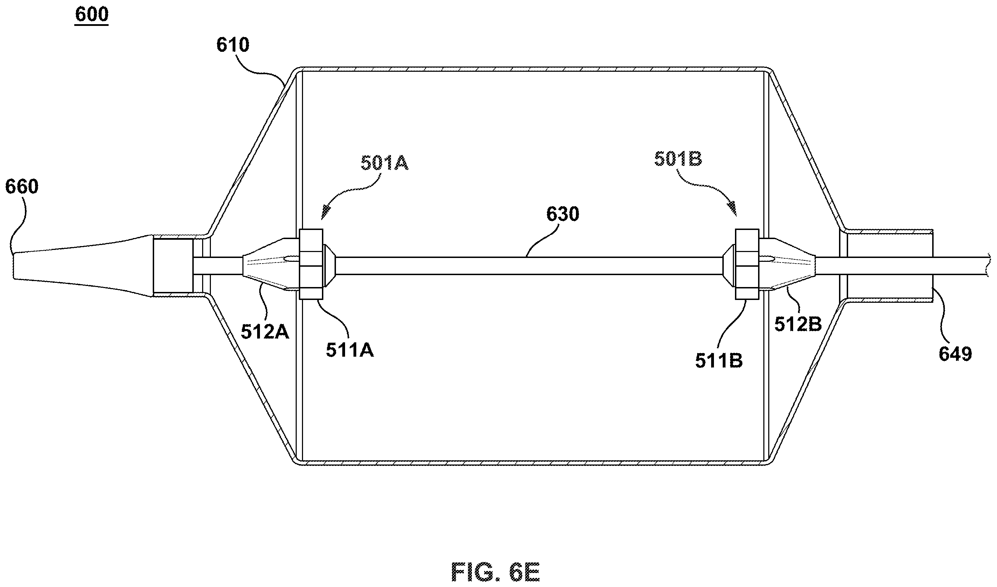

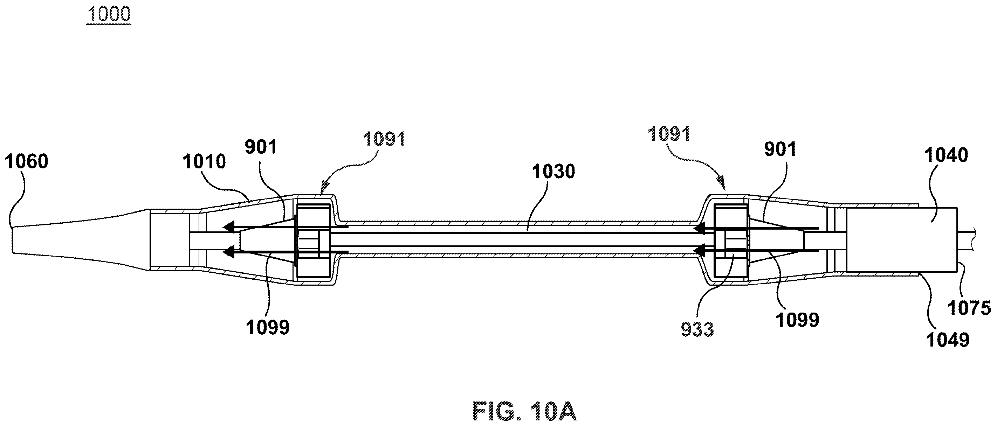

FIG. 6E illustrates a step in the assembly of the distal end of the balloon catheter 600. The outer bumpers 511A / 511B are interlocked with the inner wedges 512A / 512B. The outer bumpers 511A / 511B are stretched over respective inner wedges 512A / 512B to form the multipart retention bumpers 501. The outer bumpers 511A / 511B may be manually manipulated through the balloon 610 and/or may be manipulated via one or more tools inserted through the balloon opening 649. Such tools may include hooked or curved tubes or mandrels configured for pushing or pulling the outer bumpers 511A / 511B over the inner wedges 512A / 512B. Such tools are described in greater detail below with respect to FIGS. 10A-B. Each outer bumper 511 is slid over the corresponding inner wedge 512 from the apex 543 of the ridged tapered body 541. The core extensions 551 are radially aligned with the extension arms 542 as the outer bumpers 511A / 511B are pushed over the inner wedges 512A / 512B until they are seated on the seating hub 552. Stretching the outer bumpers 511A /511B over the inner wedges 512A / 512B causes the outer bumpers 511A / 511B to expand from the first radially unexpanded size (sufficiently narrow for entering the balloon opening 649) to the second radially expanded size (sufficiently enlarged to prevent axial migration of the prosthetic heart valve). Once seated over the seating hub 552, the outer bumpers 511A / 511B are held in place axially by the base 563 of the tapered end 562 and the base 544 of the ridged tapered body 541.

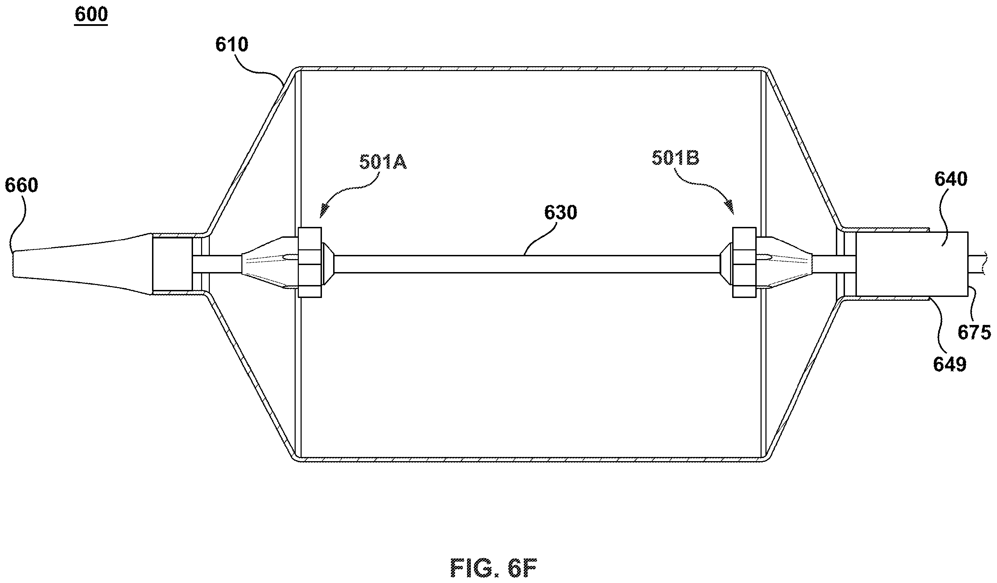

FIG. 6F illustrates a step in the assembly of the distal end of the balloon catheter 600. The outer shaft 640 is disposed over the inner shaft 630 and inserted into the balloon opening 649. The balloon 610 is secured to the outer shaft 640 via any suitable means, e.g., bonding, or adhesive. In embodiments that include heat bonding of the balloon 610 to the outer shaft 640, a support mandrel is inserted into the outer shaft 640 during the bonding step to maintain the shape of the outer shaft 640.

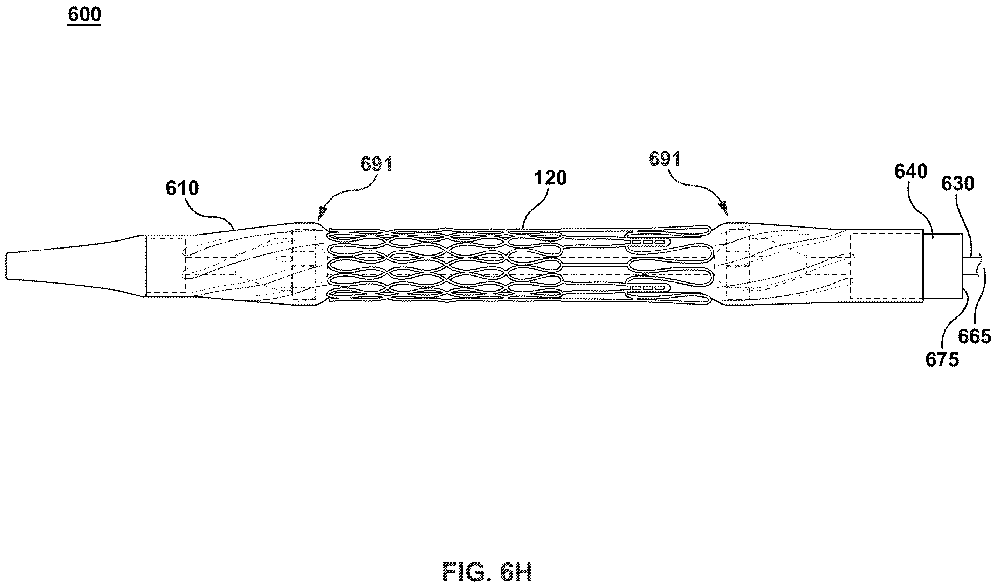

FIG. 6G illustrates a step in the assembly of the distal end of the balloon catheter 600. Air is evacuated from the balloon 610 and the balloon 610 is pleated and wrapped around the inner shaft 630. Wrapping the balloon 610 around the inner shaft 630 and the retention bumpers 501 creates stops 691 at the portions where the balloon 610 wraps over the retention bumpers 501. The stops 691 are protruding portions of the balloon 610 that prevent a prosthetic heart valve 120 crimped over the balloon 610 from migrating during delivery. FIG. 6H illustrates a step in the assembly of the distal end of the balloon catheter 600. The prosthetic heart valve 120 is crimped over the balloon 610 in the space between the stops 691 created by the multipart retention bumpers 501. Finally, the balloon catheter 600 is completed via any other processes required, including, for example, attaching the inner shaft and the outer shaft to a proximal handle.

In variations of the assembly process, various steps may take place in a different order according to different requirements. For example, in embodiments, the proximal outer bumper 511B may be loaded to the partial assembly 631 prior to insertion into the balloon 610. In another example, the outer bumpers 511 may be interlocked with the inner wedges 512 prior to bonding the balloon 610 to the distal tip 660. This may permit access to the multipart retention bumpers 501 from either end of the balloon 610. Other suitable variations may be employed without departing from the scope of this disclosure.

After completion of assembly, the balloon catheter 600 may be employed to deliver the prosthetic heart valve 120 to a treatment location. The prosthetic heart valve 120 is delivered by the balloon catheter 600 to a site of deployment. During the delivery process, the balloon catheter 600 may be guided by a guidewire passing through the guidewire lumen 665. During delivery of the prosthetic heart valve 120, the multipart retention bumpers 501 serve to maintain the axial position of the prosthetic heart valve 120 and reduce or prevent axial migration of the prosthetic heart valve 120. The multipart retention bumpers 501 combine with the wrapped balloon 610 create a stop 691 at either end of the prosthetic heart valve 120. The stop 691 applies axial force or pressure to the prosthetic heart valve 120 to maintain the position of the prosthetic heart valve 120 when it is subject to axial forces during delivery, for example, by contact with an introducer sheath. When axial force is applied to the stop 691 (and therefore to the outer bumpers 511A / 511B), the extension arms 542 of the inner wedges 512 press against the spokes 531 of the outer bumpers 511A / 511B and the base 544 of the ridged tapered body 541 presses against the inner perimeter 532 to maintain the axial position of the outer bumpers 511A / 511B. Further, the base 563 of the tapered end 562 presses against the inner perimeter 532 of the outer bumpers 511A / 511B to maintain the axial position of the outer bumper 511A / 511B, in the event that the stop 691 is subject to axial force.

Once delivered, the prosthetic heart valve 120 is deployed through operation of the balloon catheter 600. Inflation fluid, e.g., saline, contrast fluid, etc., is delivered to the balloon 610 via the inflation lumen 675 disposed between the inner shaft 630 and the outer shaft 640. The inflation fluid travels through the flow recesses 533 of the proximal inner wedges 512B, and between the extension arms 542 of the proximal inner wedges 512B and spokes 531 of the proximal outer bumpers 511B. Thus, the star shape of the retention bumpers 501 provides fluid pathways to facilitate inflation of the balloon 610. The path of the inflation fluid is shown by arrows 699 in FIG. 6G.

Due to the lack of obstruction in the fluid flow pathways, inflation fluid is permitted to flow past the multipart retention bumpers 501 along substantially longitudinal streamlines. Thus, the multipart retention bumpers 501 reduce, minimize, eliminate, or otherwise limit radial flow of inflation fluid. As used herein, substantially longitudinal streamlines refer to fluid flows having streamlines approximately parallel to a designated axis. For example, a substantially longitudinal flow in a balloon catheter may have streamlines approximately parallel to a central axis of the balloon catheter. Approximately parallel may include streamlines within 10 degrees, within 5 degrees, and/or within 1 degree of parallel. Longitudinal flows represent fluid flows along the length of the balloon catheter. Radial flows refer to fluid flows having streamlines that deviate from parallelism with a designated axis, having a significant orthogonal vector component. A streamline with a significant orthogonal vector component may deviate from parallel by 10 degrees or more. Radial flows represent flows towards or away from the axial center of the balloon catheter.

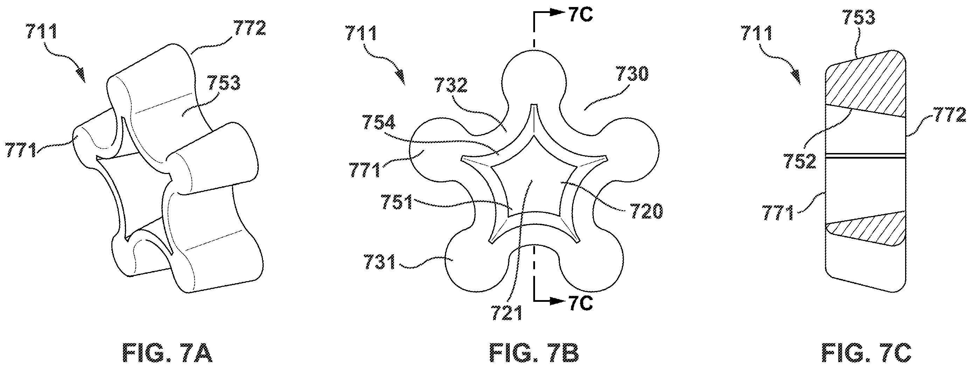

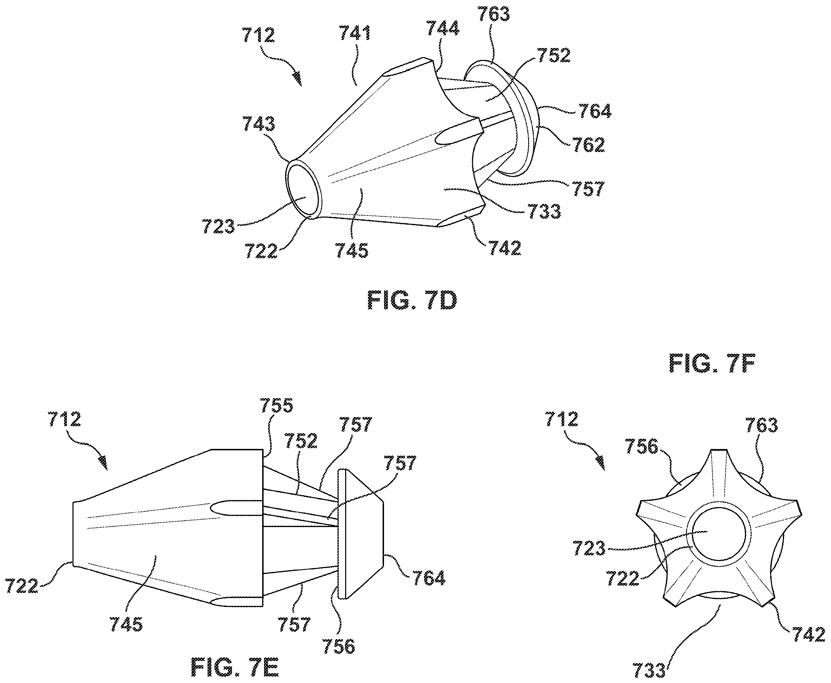

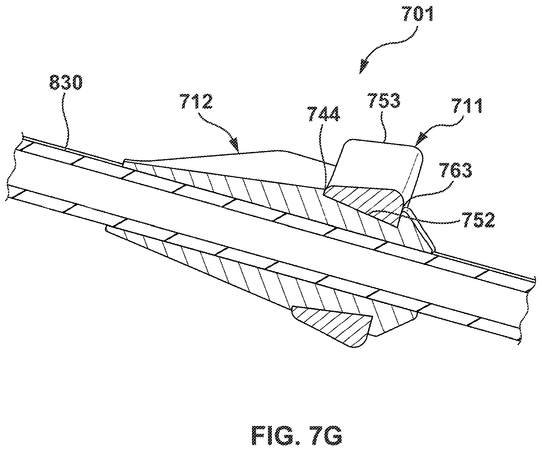

FIGS. 7A-7G illustrate multipart retention bumpers 701 consistent with embodiments hereof. FIGS. 7A-7C illustrate an outer bumper 711, FIGS. 7D-7F illustrate an inner wedge 712, and FIG. 7G is a cutaway profile view of the interlocked multipart retention bumper 701. The outer bumper 711 and the inner wedge 712 are configured such that the outer bumper 711may be stretched over the inner wedge 712, causing the radius of the outer bumper 711 to expand. Each of the outer bumper 711 and the inner wedge 712 have a star shape, with an inner core or hub and extensions, arms, or spokes extending radially.

The multipart retention bumper 701 includes the inner wedge 712 and the outer bumper 711. The inner wedge 712 and outer bumper 711 are configured such that, when interlocked, the maximum diameter of the outer bumper 711 (and thus the maximum diameter of the multipart retention bumpers 701) expands from a first radially unexpanded size to a second radially expanded size, wherein the first radially unexpanded size is selected to fit within the neck of a balloon during assembly of a balloon catheter and the second radially expanded size is selected to permit the interlocked multipart retention bumper 701 to maintain the axial position of a prosthetic heart valve crimped to the balloon, as discussed in greater detail below.

FIGS. 7A-7C illustrate the outer bumper 711. FIG. 7A is a perspective view of the outer bumper 711, FIG. 7B is a plan view of the outer bumper 711 looking at an apex surface 771 of the outer bumper 711, and FIG. 7C is a profile view of the outer bumper 711. The outer bumper 711 is formed from any suitable flexible material, including elastic materials, such as polymers, elastomers, thermoplastic elastomers, PTFE, PS, PET, etc. and is configured to expand. In embodiments, the outer bumper 711 may be formed from a thermoplastic elastomer (TPE) such as Pebax^{®} 55.

In profile, the outer bumper 711 is tapered from an apex surface 771 to a base surface 772. The outer bumper 711 includes a spoked perimeter 730 surrounding a hollow core 720. The spoked perimeter 730 includes a plurality of spokes 731 that project outward from an inner perimeter 732. The spoked perimeter 730 surrounds the hollow core 720, which is an empty interior space including a central core 721 and core extensions 751.

The spoked perimeter includes an outer wall 753 and an inner wall 754. The outer wall 753 is tapered and defines a profile that is narrower in effective diameter at the apex surface 771 of the outer bumper 711 and wider in effective diameter at the base surface 772 of the outer bumper, where the apex surface 771 is opposite the base surface 772. The tapered profile of the outer wall 753 is best seen in FIG. 7C. The inner wall 754 defines the central core 721 and the core extensions 751. The inner wall 754 is tapered and defines a profile that is narrower in effective diameter at the side of the base surface 772 of the outer bumper 711 and wider in effective diameter at the side of the apex surface 771 of the outer bumper 711. Thus, the inner wall 754 and outer wall 753 are tapered in opposite directions. This feature is further illustrated by the cutaway assembly view in FIG. 7G.

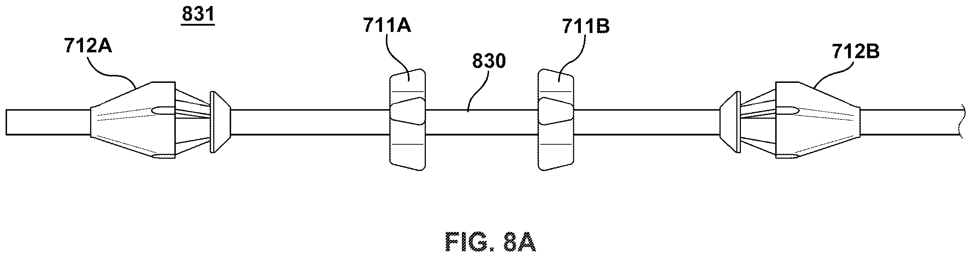

The inner wedge 712 includes a central hub 722 surrounding an interior cavity 723. FIG. 7D is a perspective view of inner wedge 712, FIG. 7E is a profile view of inner wedge 712, and FIG. 7F is a plan view of inner wedge 712. As shown, e.g., in FIGS. 8A-8H, the interior cavity 723 is sized and configured suitably to accommodate an inner shaft of a balloon catheter, such as an inner shaft 830. The inner wedge 712 further includes three sections disposed over the central hub 722. The first section, disposed at one end of the central hub 722, is a ridged tapered body 741 having a star shape. The second section, disposed in a middle portion of the central hub 722, is a seating hub 752. The third section disposed at the end of the central hub 722 opposite the ridged tapered body 741, is a tapered end 762. The interior cavity 723 runs axially through the ridged tapered body 741, the seating hub 752, and the tapered end 762.

The ridged tapered body 741 includes a tapered base 745, extension arms 742, an apex 743 at the end of the inner wedge 712, and a base 744 at the intersection between the ridged tapered body 741 and the seating hub 752. The star shape of the ridged tapered body 741 is defined by the tapered base 745 and the extension arms 742. The diameter of the apex 743 is smaller than the diameter of the base 744. Extension arms 742 extend radially from the tapered base 745. The extension arms 742 have a taper, such that they extend further from the tapered base 745 at the base 744 than at the apex 743. Accordingly, the extension arms 742 define a largest effective diameter of the inner wedge 712 at the base 744 of the ridged tapered body 741. The empty spaces between the extension arms 742 are flow recesses 733. The flow recesses 733 extend from the tapered base 745 to a diameter defined by extension arms 742.

The inner wedge 712 further includes a seating hub 752 adjacent to the base 744 of the ridged tapered body 741. The seating hub 752 is hollow, with the hollow occupied by the interior cavity 723. The seating hub 752 is a tapered base with a wider base end 755 at the intersection with the ridged tapered body 741 and a narrower apex end 756 at the intersection with the tapered end 762. At the base end 755, the seating hub 752 is greater than the diameter of the interior cavity 723 and less than the diameter of the base 744 of the ridged tapered body 741. At the apex end 756, the seating hub 752 is greater than the diameter of the interior cavity 723 and less than the diameter of the base 763 of the tapered end 762. Thus, the base 744 of the ridged tapered body 741 forms a flange or stop at one side of the seating hub 752 and the base 763 of the tapered end 762 forms a flange or stop at the other side of the seating hub 752. Further, the seating hub 752 includes stabilization fins 757. The stabilization fins 757 extend radially from the seating hub 752 with a taper having a narrow end at the apex end 756 of the seating hub 752 and a wider end at the base end 755 of the seating hub 752. Thus, the stabilization fins 757 are tapered in the same direction as the taper of the seating hub 752, extending to a larger effective diameter at the base end 755 of the seating hub 752. In embodiments, the radial arrangement of the stabilization fins 757 may correspond with the radial arrangement of the extension arms 742. For example, the number of stabilization fins 757 may be the same as the number of the extension arms 742, and the stabilization fins 757 and the extension arms 742 may be radially aligned.

The taper of the seating hub 752 and the taper of the stabilization fins 757 is selected to correspond to the taper of the inner wall 754 of the outer bumper 711 when the outer bumper 711 is elastically stretched or deformed to interlock with the inner wedge 712. When the outer bumper 711 is interlocked with the inner wedge 712, as shown in FIG. 7G, the stabilization fins 757 extend into the core extensions 751 of the hollow core 720 of the outer bumper 711. Thus, the shape of the inner wall 754 is configured to conform to the shape of the seating hub 752 including the stabilization fins 757 when in the inner wedge 712 and the outer bumper 711 are in an interlocked configuration. Further, the outer diameter of the seating hub 752 is larger than the inner diameter of the inner wall 754, such that, when the outer bumper 711 is in an interlocked configuration with the inner wedge 712, the outer bumper 711 remains stretched and provides a greater effective diameter, i.e., a radially expanded size, to the multipart retention bumper 701 than is provided by either the inner wedge 712 or the outer bumper 711 in a non-interlocked configuration.

The inner wedge 712 further includes a tapered end 762. The tapered end 762 is a tapered shape with a base 763 adjacent the seating hub 752 and an apex 764 at the end of the inner wedge 712. The base 763 of the tapered end 762 has a diameter greater than the diameter of the seating hub 752. Thus, the base 763 of the tapered end 762 forms a flange or stop at a side of the seating hub 752 opposite the ridged tapered body 741. The tapered profile of the tapered end 762, in combination with the taper of the inner wall 754 of the outer bumper 711, serves to facilitate the interlock of the outer bumper 711 and the inner wedge 712 in the multipart retention bumper 701. During an interlock procedure, the outer bumper 711 may be stretched around the inner wedge 712 as it is advanced over the tapered end 762 to rest in the seating hub 752. The combination of tapers of the inner wall 754 and the tapered end 762 provide outward radial force to the outer bumper 711 as it is advanced axially, causing the outer bumper 711 to stretch.

The inner wedge 712 is formed from a suitable rigid material, including suitable plastics or polymers, including high density polyethylene (HDPE), TPE, Pebax^{®} and any other suitable rigid material. In embodiments, the material of the inner wedge 712 is selected so as to be substantially non-compressible compression during conditions expected to accompany manufacture, assembly, or use. As used herein, substantially non-compressible during conditions expected to accompany manufacture, assembly, or use means that the inner wedges 712 show less than 5%, less than 3%, and/or less than 1% strain when subject to forces or stresses common during manufacture, assembly, and/or use. For example, a suitable durometer may be in excess of 60D, in excess of 65D, in excess of 70D, or in excess of 75D.

FIG. 7G illustrates the multipart retention bumper 701 interlocked, with the outer bumper 711 disposed over the seating hub 752 of the inner wedge 712. Once seated in the seating hub 752, the position of the outer bumper 711 is maintained both radially and axially by the mating of the features of the seating hub 752 with the features of the outer bumper 711. The stabilization fins 757 mate with the core extensions 751 to prevent rotation of the outer bumper 711 with respect to the inner wedge 712. As discussed above, the radial arrangement of the stabilization fins 757 may correspond to the radial arrangement of the extension arms 742. The stabilization fins 757 mate with the core extensions 751, which correspond radially with the spokes 731. Thus, in the interlocked configuration, the stabilization fins 757, the spokes 731, and the extension arms 742 are all radially aligned. Thus, preventing rotation of the outer bumper 711 with respect to the inner wedges 712 serves to maintain radial alignment between the spokes 731 and the extension arms 742. The base 744 of the ridged tapered body 741 serves as a flange or stop to arrest axial movement of the outer bumper 711 with respect to the inner wedge 712 in one direction. The base 763 of the tapered end 762 serves as a flange or stop to arrest axial movement of the outer bumper 711 with respect to the inner wedge 712 in the other direction.

The outer bumper 711 is configured to stretch, i.e., elastically deform, around the inner wedge 712 and to be disposed on the seating hub 752 of the inner wedge 712 when the outer bumper 711 and the inner wedge 712 are in an interlocked configuration. As discussed above, the seating hub 752 of the inner wedge 712 has a diameter greater than that of the inner wall 754 such that, when the outer bumper 711 is disposed on the seating hub 752, the outer bumper 711 remains in a stretched configuration. The stretched configuration of the outer bumper 711 causes an increase in its effective diameter.

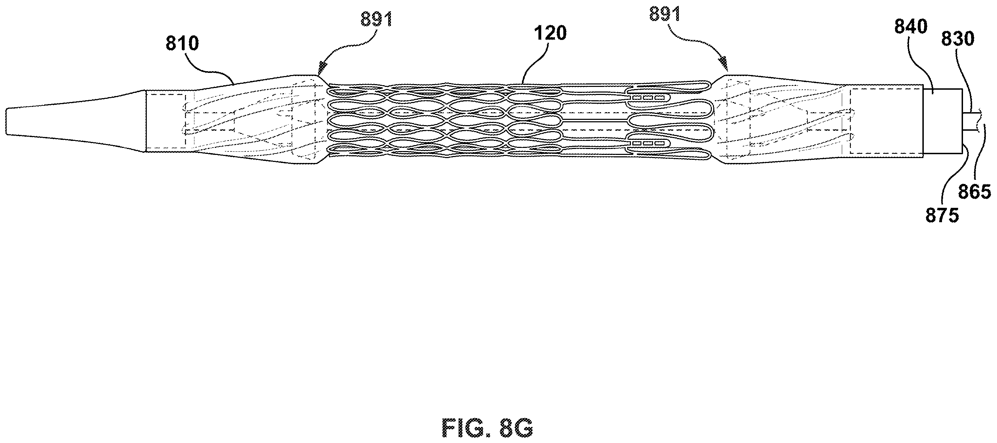

When disposed over the seating hub 752, the spokes 731 of the outer bumper 711 have an increased effective diameter due to stretching caused by the diameter of the seating hub 752. The outer bumper 711 may be disposed on the seating hub 752 via stretching over either the tapered end 762 or over the ridged tapered body 741. As discussed above, stretching over the tapered end 762 is facilitated by the tapers of the tapered end 762 and the inner wall 754. Once seated, the spokes 731 of the outer bumper 711 are arranged in radial alignment with the extension arms 742 of the inner wedge 712. As discussed above, this radial alignment is maintained by the stabilization fins 757. This arrangement allows inflation fluid to flow between the extension arms 742 (in the flow recesses 733) and between the spokes 731, without significant obstruction. Further, the extension arms 742 of the inner wedge 712 provide lateral support for the spokes 731 of the outer bumper 711. These features are discussed in greater detail below with respect to FIG. 8G.

In embodiments, each of the inner wedges 712 and outer bumpers 711 are configured with maximum diameters between approximately 0.1 inches and 0.25 inches, between 0.15 and 0.22 inches, between 0.19 and 0.21 inches, or approximately 0.2 inches. Such sizes correspond to a first radially unexpanded size and may be suitable for entry into the opening of a balloon of a balloon catheter, as discussed in greater detail below with respect to FIGS. 8A-8H. In embodiments, the multipart retention bumpers 701 may have a maximum diameter between 0.25 inches and 0.35 inches, between 0.27 and 0.33 inches, between 0.29 and 0.31 inches, or approximately 0.3 inches after the inner wedges 712 and the outer bumpers 711 are interlocked. Such sizes correspond to a second radially expanded size and are suitable for prevention of axial migration of a prosthetic heart valve located between the inner wedges 712 and the outer bumpers 711 when the prosthetic heart valve is subject to force.

Prior to stretching over the inner wedge 712, the outer bumper 711 has an effective diameter at the first radially unexpanded size smaller than a balloon opening. After stretching, the outer bumper 711 has an effective diameter at the second radially expanded size great enough to retain a prosthetic heart valve. Seating the outer bumper 711 on the seating hub 752 of the inner wedge 712 causes the effective diameter of the outer bumper 711 to expand from a first radially unexpanded size to a second radially expanded size.

FIGS. 8A-8G illustrate a balloon catheter assembly process including the multipart retention bumpers 701, as formed from inner wedges 712 and outer bumpers 711. FIGS. 8A-8G illustrate an assembly process employing the multipart retention bumpers 701 for example purposes only. The assembly process of FIGS. 8A-8G is by way of example only, and balloon catheters including multipart retention bumpers may be assembled using different steps in a different order without departing from the scope of the present disclosure.

FIG. 8A illustrates a partial assembly 831 prepared for balloon catheter assembly. The partial assembly 831, at this stage of balloon catheter assembly, includes the inner shaft 830 and distal and proximal inner wedges 712A / 712B secured thereto. Over molding of the inner wedges 712 onto the inner shaft 830 may be selected to provide a sure connection throughout usage of the balloon catheter, facilitate manufacturing, and insure accurate placement of the inner wedges 712 on the inner shaft 830. In further embodiments, the inner wedges 712 may be secured to the inner shaft 830 via other techniques, such as by bonding and/or adhesive. The partial assembly further includes the distal and proximal outer bumpers 711A/712B disposed over the inner shaft 830. The distal and proximal outer bumpers 711A/712B are not secured to the inner shaft 830 at this stage. The distal and proximal outer bumpers 711A/712B may be disposed over the inner shaft 830 either prior to securing the distal and proximal inner wedges 712A / 712B or after securing the distal and proximal inner wedges 712A / 712B.

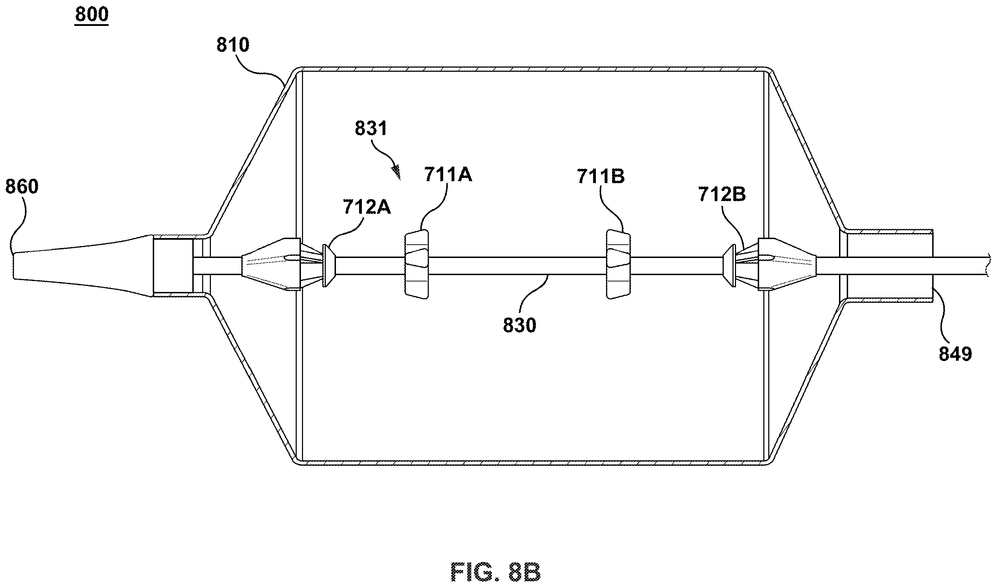

FIG. 8B illustrates a step in the assembly of the distal end of the balloon catheter 800. The balloon 810 and distal tip 860 are added to the partial assembly 831. The inner shaft 830 is secured to the distal tip 860 by any suitable means, e.g., adhesive, heat bonding, etc. The partial assembly 831, including the inner shaft 830, the distal tip 860, inner wedges 712, and the outer bumpers 711, is slid into the balloon 810 through the proximal balloon opening 849 of the balloon 810. The balloon 810 is then secured to the distal tip via any suitable means, e.g., via heat bonding. As discussed above, the inner wedges 712 and the outer bumpers 711 are sized with an effective diameter at the first radially unexpanded size, sufficiently narrow for passing through the proximal balloon opening 849 of the balloon 810 without compression of any type.

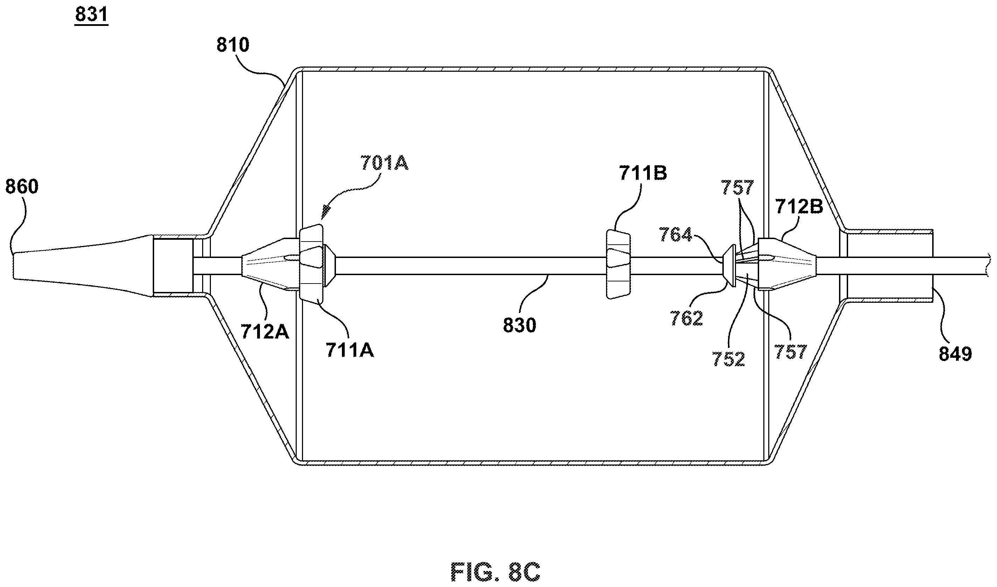

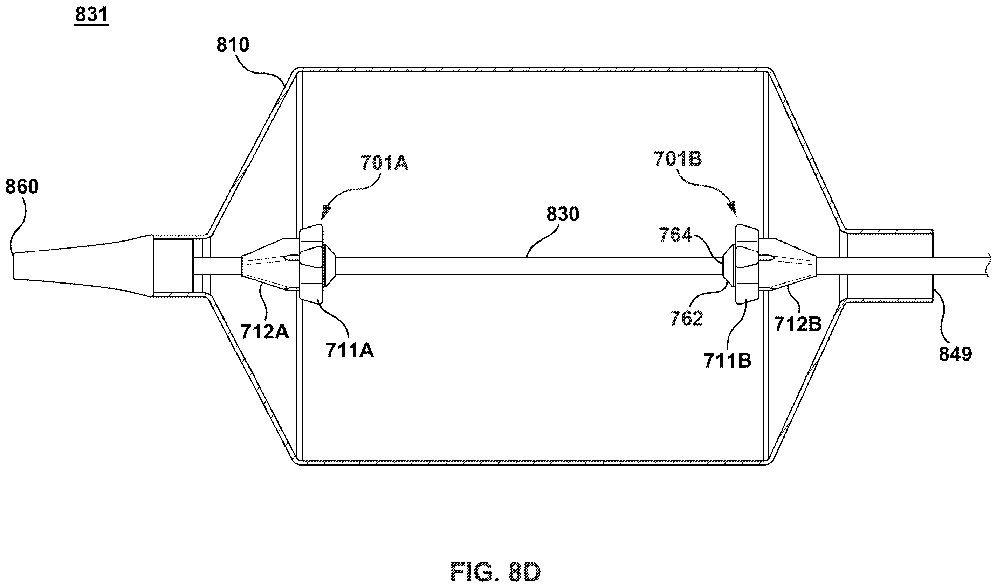

FIG. 8C and 8D illustrate steps in the assembly of the distal end of the balloon catheter 800. The outer bumpers 711 are interlocked with the inner wedges 712 to form the retention bumpers 701. The distal outer bumper 711A is stretched over the distal wedge 712A to form the distal multipart retention bumper 701A, as shown in FIG. 8C. The distal outer bumper 711B is stretched over the distal wedge 712B to form the distal multipart retention bumper 701B, as shown in FIG. 8D.

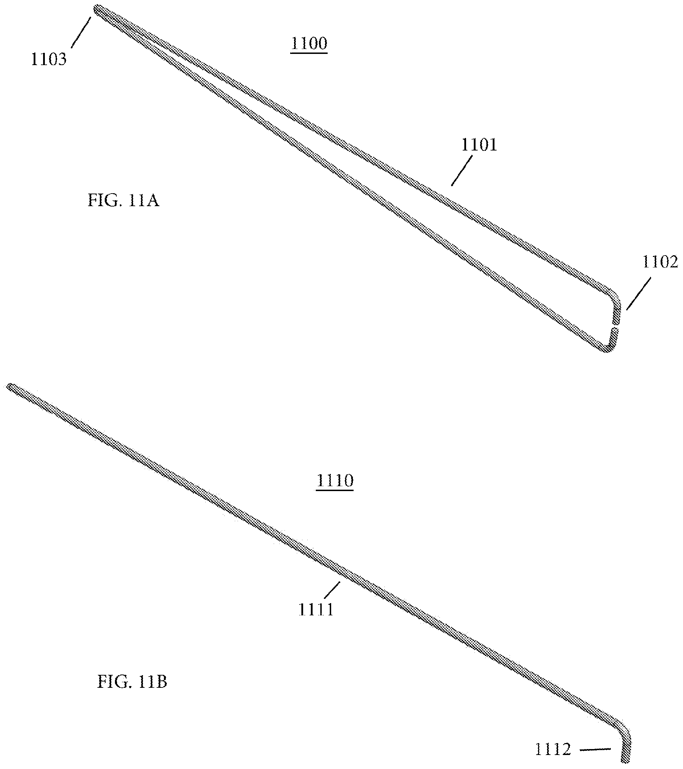

The outer bumpers 711 may be manually manipulated through the balloon 810 and/or may be manipulated via one or more tools inserted through the balloon opening 849. Such tools may include hooked or curved tubes or mandrels configured for pushing or pulling the outer bumpers 711 over the inner wedges 712. Such tools are described in greater detail below with respect to FIGS. 11A-11B. Each outer bumper 711 is slid over the corresponding inner wedge 712 from the apex 764 of the tapered end 762. The core extensions 751 of the outer bumpers 711 are radially aligned with the stabilization fins 757 of the inner wedges 712 as the outer bumpers 711 are pushed over the inner wedges 712 until they are seated on the seating hub 752. Stretching the outer bumpers 711 over the inner wedges 712 causes the outer bumpers 711 to expand from the first radially unexpanded size (sufficiently narrow for entering the balloon opening 849) to the second radially expanded size (sufficiently enlarged to prevent axial migration of the prosthetic heart valve). The tapers of the inner wall 754 of the outer bumpers 711 and the tapered end 762 of the inner wedges 712 facilitate the stretching of the outer bumpers 711. Once seated over the seating hub 752, the outer bumpers 711 are held in place axially by the base 763 of the tapered end 762 and the base 744 of the ridged tapered body 741. The outer bumpers 711 are prevented from rotating with respect to the inner wedges 712 by the stabilization fins 757.

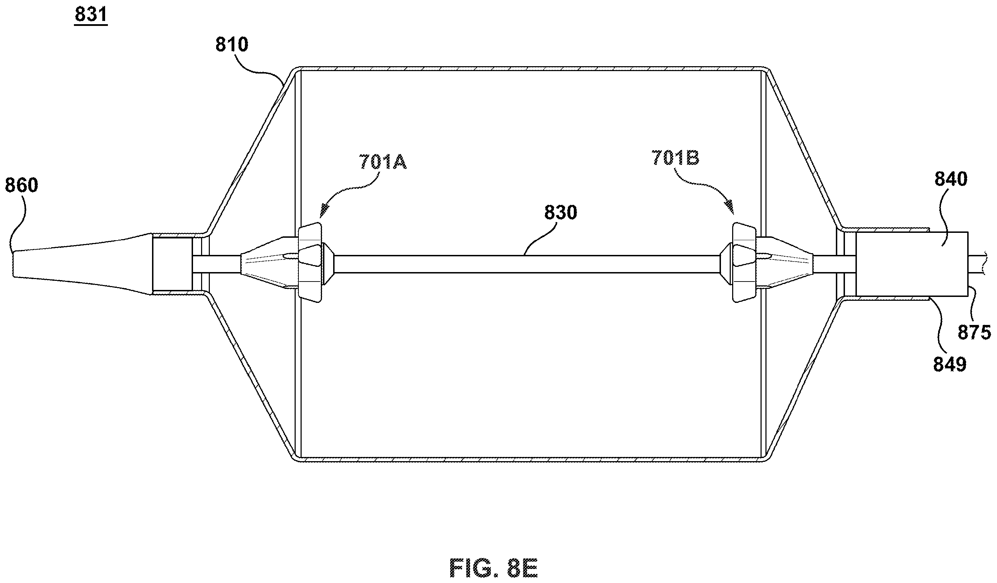

FIG. 8E illustrates a step in the assembly of the distal end of the balloon catheter 800. The outer shaft 840 is disposed over the inner shaft 830 and inserted into the balloon opening 849. The balloon 810 is secured to the outer shaft 840 via any suitable means, e.g., bonding, or adhesive. In embodiments that include heat bonding of the balloon 810 to the outer shaft 840, a support mandrel is inserted into the outer shaft 840 during the bonding step to maintain the shape of the outer shaft 840.

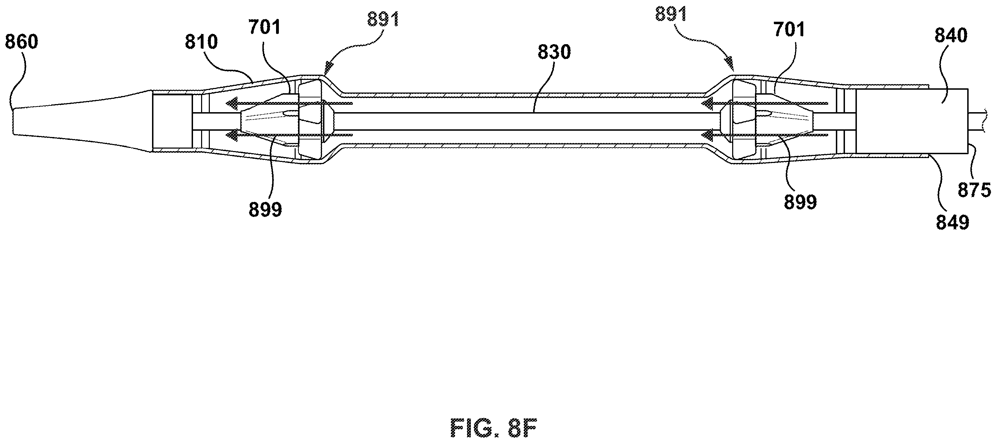

FIG. 8F illustrates a step in the assembly of the distal end of the balloon catheter 600. Air is evacuated from the balloon 810 and the balloon 810 is pleated and wrapped around the inner shaft 830.

FIG. 8G illustrates a step in the assembly of the distal end of the balloon catheter 800. The prosthetic heart valve 120 is crimped over the pleated and folded balloon 810. Wrapping the balloon 810 around the inner shaft 830 and the retention bumpers 701 creates stops 891 at the portions where the balloon 810 wraps over the retention bumpers 701. The stops 891 are protruding portions of the balloon 810 that prevent a prosthetic heart valve 120 crimped over the balloon 810 from migrating during delivery. The prosthetic heart valve 120 is crimped over the balloon 810 in the space between the stops 891 created by the multipart retention bumpers 701. Finally, the balloon catheter 800 is completed via any other processes required, including, for example, attaching the inner shaft and the outer shaft to a proximal handle.

In variations of the assembly process, various steps may take place in a different order according to different requirements. For example, the outer bumpers 711 may be interlocked with the inner wedges 712 prior to bonding the balloon 810 to the distal tip 860. This may permit access to the multipart retention bumpers 701 from either end of the balloon 810. Other suitable variations may be employed without departing from the scope of this disclosure.

After completion of assembly, the balloon catheter 800 may be employed to deliver the prosthetic heart valve 120 to a treatment location. After completion of assembly, the balloon catheter 800 may be employed to deliver the prosthetic heart valve 120 to a treatment location. The prosthetic heart valve 120 is delivered by the balloon catheter 800 to a site of deployment. During the delivery process, the balloon catheter 800 may be guided by a guidewire passing through the guidewire lumen 865. During delivery of the prosthetic heart valve 120, the multipart retention bumpers 701 serve to maintain the axial position of the prosthetic heart valve 120 and reduce or prevent axial migration of the prosthetic heart valve 120. The multipart retention bumpers 701 combine with the wrapped balloon 810 create a stop 891 at either end of the prosthetic heart valve 120. The stop 891 applies axial force or pressure to the prosthetic heart valve 120 to maintain the position of the prosthetic heart valve 120 when it is subject to axial or other forces during delivery, for example, by contact with an introducer sheath. When axial force is applied to the stop 891 (and therefore to the outer bumpers 711), the extension arms 742 of the inner wedges 712 press against the spokes 731 of the outer bumpers 711 and the base 744 of the ridged tapered body 741 presses against the inner perimeter 732 to maintain the axial position of the outer bumpers 711. Further, the base 763 of the tapered end 762 presses against the inner perimeter 732 of the outer bumpers 711 to maintain the axial position of the outer bumper 711, in the event that the stop 891 is subject to axial force.

Once delivered, the prosthetic heart valve 120 is deployed through operation of the balloon catheter 800. Inflation fluid, e.g., saline, contrast fluid, etc., is delivered to the balloon 810 via the inflation lumen 875 disposed between the inner shaft 830 and the outer shaft 840. The inflation fluid travels through the flow recesses 733 of the inner wedges 712, and between the extension arms 742 of the inner wedges 712 and spokes 731 of the outer bumpers 711. Thus, the star shape of the retention bumpers 701 provides fluid pathways to facilitate inflation of the balloon 810. The star shape of the retention bumpers 701 and the flow recesses 733 are best illustrated in FIG. 7F. The path of the inflation fluid is shown by arrows 899 in FIG. 8F.

Due to the lack of obstruction in the fluid flow pathways, inflation fluid is permitted to flow past the retention bumpers 701 along substantially longitudinal streamlines. Thus, the retention bumpers 701 reduce, minimize, eliminate, or otherwise limit radial flow of inflation fluid. As used herein, substantially longitudinal streamlines refer to fluid flows having streamlines approximately parallel to a designated axis. For example, a substantially longitudinal flow in a balloon catheter may have streamlines approximately parallel to a central axis of the balloon catheter. Approximately parallel may include streamlines within 10 degrees, within 5 degrees, and/or within 1 degree of parallel. Longitudinal flows represent fluid flows along the length of the balloon catheter. Radial flows refer to fluid flows having streamlines that deviate from parallelism with a designated axis, having a significant orthogonal vector component. A streamline with a significant orthogonal vector component may deviate from parallel by 10 degrees or more. Radial flows represent flows towards or away from the axial center of the balloon catheter.

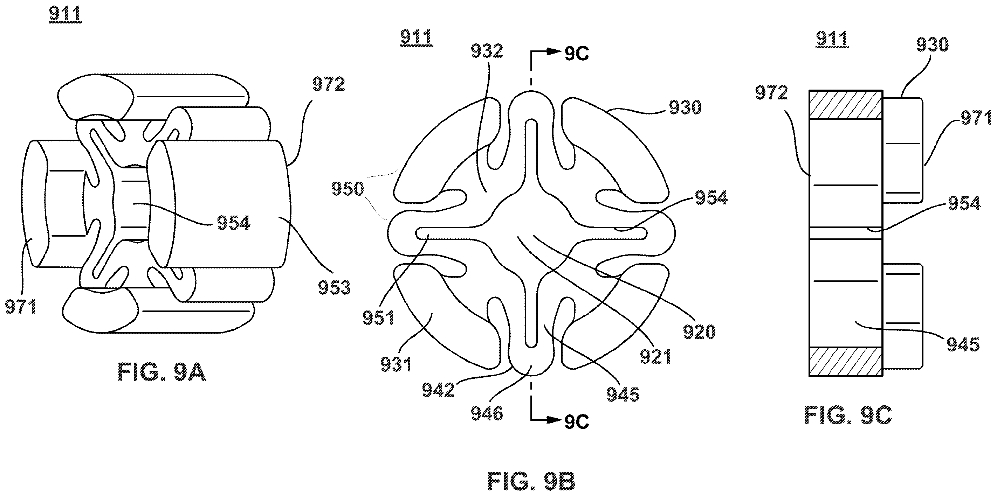

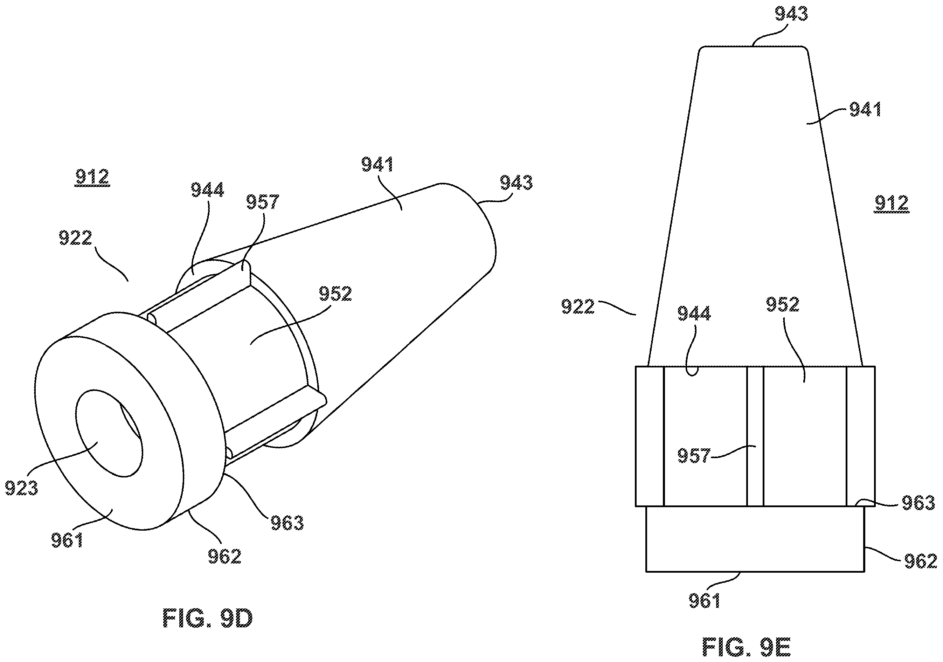

FIGS. 9A-9H illustrate multipart retention bumpers 901 consistent with embodiments hereof. The multipart retention bumpers 901 include outer bumpers 911 (FIGS. 9A-9C) and inner wedges 912 (FIGS. 9D-9G). FIGS. 9A-9C illustrate the outer bumper 911. FIG. 9A is a perspective view of the outer bumper 911, FIG. 9B is a plan view of the outer bumper 911 looking at a valve abutment surface 971 of the outer bumper 911, and FIG. 9C is a cross-section profile view of the outer bumper 911. The outer bumper 911 is formed from any suitable flexible material, including elastic materials, such as polymers, elastomers, thermoplastic elastomers, PTFE, PS, PET, etc. and is configured to expand. In embodiments, the outer bumper 911 may be formed from a thermoplastic elastomer (TPE) such as Pebax^{®} 55.

In profile, the outer bumper 911 is substantially straight-sided from the valve abutment surface 971 to a base surface 972. The outer bumper 911 includes a spoked perimeter 930 surrounding a hollow core 920. The spoked perimeter 930 includes a plurality of spokes that form valve stops 931 and perimeter extensions 942. The valve stops 931 are located on an outer wall 953 of an inner perimeter 932. The inner perimeter 932 further includes the perimeter extensions 942. The perimeter extensions 942 are loops of material that extend out from the inner perimeter 932 and include arms 945 that extend from the inner perimeter 932 and are joined at their ends by hinges 946. Between each pair of perimeter extensions 942 is one of the valve stops 931, a solid portion of material that extends outward from the inner perimeter 932. One longitudinal end or side of each valve stop 931 forms a portion of the base surface 972 of the outer bumper 911 while the opposing longitudinal end or side of each valve stop 931 forms a portion of the abutment surface 971. The valve stops 931 extend longitudinally away from the spoked perimeter 930 in a direction parallel to the central axis of the outer bumper 911 such that the abutment surfaces 971 are disposed on a plane different than a plane of the spoked perimeter. The spoked perimeter 930 expands at least partially through a hinging action of the hinges 946 at the ends of the arms 945. When the perimeter extensions 942 hinge open, arms 945 of each perimeter extension 942 spread apart, causing an expansion of the spoked perimeter 930. The spoked perimeter 930 surrounds the hollow core 920, which is an empty interior space including a central core 921 and core extensions 951. The core extensions 951 are defined by the perimeter extensions 942.

As shown in FIG. 9B, the spoked perimeter 930 of the outer bumper 911 is generally star-shaped, with the valve stops 931 and the perimeter extensions 942 acting as the arms of the star. The hollow core 920 is also star-shaped, with the core extensions 951 acting as the arms of the star.

The spoked perimeter 930 includes an outer wall 953 defining an external surface of the spoked perimeter 930 and an inner wall 954 defining an internal surface and surrounding the hollow core 920 of the spoked perimeter 930. The outer wall 953 is substantially straight-sided from the valve abutment surface 971 to the base surface 972. The inner wall 954 defines the central core 921 and the core extensions 951. The inner wall 954 is substantially straight-sided from the valve abutment surface 971 to the base surface 972. Thus, the inner wall 954 and outer wall 953 are substantially parallel to one another. As used herein, substantially parallel refers to surfaces that are parallel to within approximately 5° of one another.

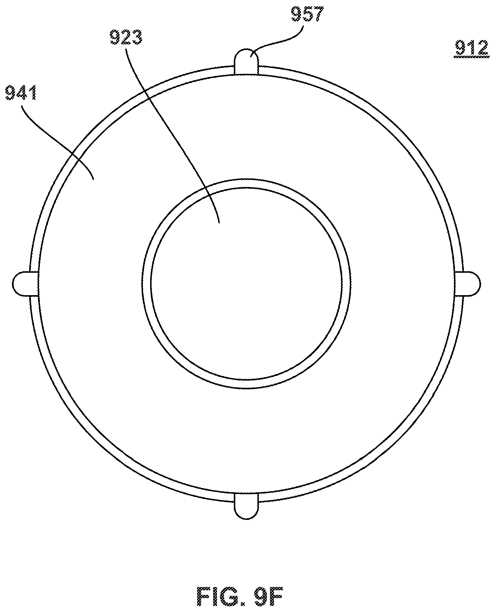

FIGS. 9D-9F illustrate the inner wedge 912. FIG. 9D is a perspective view of inner wedge 912, FIG. 9E is a cross section profile view of inner wedge 912, and FIG. 9F is a plan view of inner wedge 912. The inner wedge 912 includes a central body portion 922 surrounding an interior cavity 923. The interior cavity 923 is sized and configured suitably to accommodate an inner shaft of a balloon catheter, such as an inner shaft 1030, as discussed below. The central body portion 922 of the inner wedge 912 includes three sections. The first section, disposed at one end of the central body portion 922, is a tapered body 941. The second section, disposed in a middle portion of the central body portion 922, is a seating hub 952. The third section disposed at the end of the central body portion 922 opposite the tapered body 941, is a stop portion 962. The interior cavity 923 runs axially through the tapered body 941, the seating hub 952, and the stop portion 962.

The tapered body 941 has axial symmetry and includes a base 944 and an apex 943. The base 944 is at the intersection between the tapered body 941 and the seating hub 952. The apex 943 is at an end of the inner wedge 912. The base 944 and the apex 943 are each circular, with the base 944 having a larger diameter than the apex 943. The sides of the tapered body 941 are smooth and taper from the base 944 to the apex 943. The tapered body 941 surrounds a cavity forming a portion of the interior cavity 923.

The tapered profile of the tapered body 941 serves to facilitate the interlock of the outer bumper 911 and the inner wedge 912 in the multipart retention bumper 901. During an interlock procedure, the outer bumper 911 may be expanded around the inner wedge 912 as it is advanced over the tapered body 941 to rest in the seating hub 952. The taper of the tapered body 941 provides outward radial force to the outer bumper 911 as it is advanced axially, causing the outer bumper 911 to expand.

The stop portion 962 is a cylindrical shape located adjacent the seating hub 952, opposite the tapered body 941. The stop portion 962 has axial symmetry and has an outer diameter greater than the outer diameter of the seating hub 952. The stop portion 962 includes an interior cavity forming a portion of the interior cavity 923. The stop portion 962 has an interior face 963 facing the seating hub 952 and an exterior face 961 that forms the end face of the inner wedge 912.

The seating hub 952 is located between the tapered body 941 and the stop portion 962. The seating hub 952 includes a cavity which, together with the cavities of the stop portion 962 and the tapered body 941 forms the interior cavity 923. The seating hub 952 is cylindrical. The seating hub 952 has an outer diameter greater than the outer diameter of the interior cavity 923 and smaller than the outer diameter of the base 944 of the tapered body 941 and smaller than the outer diameter of the stop portion 962. Thus, the base 944 of the tapered body 941 forms a flange or stop at one longitudinal end of the seating hub 952 and the interior face 963 of the stop portion 962 forms a flange or stop at the other longitudinal end of the seating hub 952. Further, the seating hub 952 includes stabilization fins 957 extending radially from the seating hub 952. With the exception of the stabilization fins 957, the seating hub 952 has axial symmetry. The radial arrangement of the stabilization fins 957 corresponds with the radial arrangement of the core extensions 951 and perimeter extensions 942 of the outer bumper 911. For example, the number of stabilization fins 957 may be the same as the number of the perimeter extensions 942, and the stabilization fins 957 and the perimeter extensions 942 may be radially aligned when the inner wedge 912 and the outer bumper 911 are interlocked.

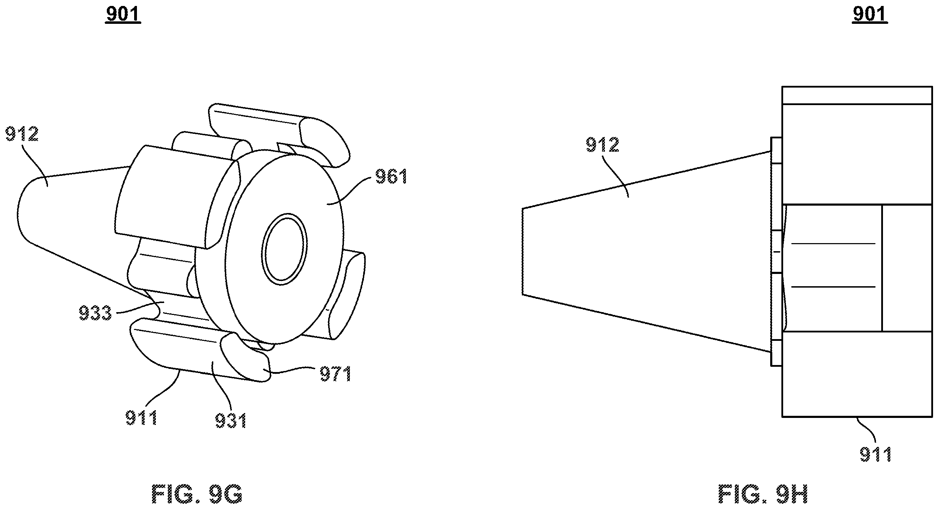

When the outer bumper 911 is interlocked with the inner wedge 912, as shown in FIGS. 9G and 9H, the stabilization fins 957 extend into the core extensions 951 of the hollow core 920 of the outer bumper 911. In this configuration, the stabilization fins 957 are surrounded by the perimeter extensions 942. The shape of the inner wall 954 is configured to conform to the cylindrical shape of the seating hub 952 including the stabilization fins 957 when the inner wedge 912 and the outer bumper 911 are in an interlocked configuration. Further, the outer diameter of the seating hub 952 is larger than the inner diameter of the inner wall 954, such that, when the outer bumper 911 is in an interlocked configuration with the inner wedge 912, the outer bumper 911 remains expanded and provides a greater effective diameter, i.e., a radially expanded size, to the multipart retention bumper 901 than is provided by either the inner wedge 912 or the outer bumper 911 in a non-interlocked configuration.

The inner wedge 912 is formed from a suitable rigid material, including suitable plastics or polymers, including high density polyethylene (HDPE), TPE, Pebax^{®} and any other suitable rigid material. In embodiments, the material of the inner wedge 912 is selected so as to be substantially non-compressible during conditions expected to accompany manufacture, assembly, or use. As used herein, substantially non-compressible during conditions expected to accompany manufacture, assembly, or use means that the inner wedges 912 show less than 5%, less than 3%, and/or less than 1% strain when subject to forces or stresses common during manufacture, assembly, and/or use. For example, a suitable durometer may be in excess of 60D, in excess of 65D, in excess of 70D, or in excess of 75D. In embodiments, the inner wedge 912 may include a radiopaque doping agent. The inclusion of a radiopaque doping agent in the material or materials of the inner wedge 912 does not compromise the performance of the inner wedge 912. The radiopaque doping agent in the inner wedge 912 improves the visibility of the inner wedge 912 during clinical imaging operations, such as fluoroscopy. Accordingly, the radiopacity of the inner wedges 912 may be used by a doctor or other operator to assist in positioning of the prosthetic valve held in place by the retention bumpers 901.

FIGS. 9G and 9H illustrate the multipart retention bumper 901 interlocked, with the outer bumper 911 disposed over the seating hub 952 of the inner wedge 912. Once seated in the seating hub 952, the position of the outer bumper 911 is maintained both radially and axially by the mating of the features of the seating hub 952 with the features of the outer bumper 911. The stabilization fins 957 fit into the core extensions 951 to mate with the perimeter extensions 942 to prevent rotation of the outer bumper 911 with respect to the inner wedge 912. As discussed above, the radial arrangement of the stabilization fins 957 corresponds to the radial arrangement of the perimeter extensions 942. Thus, in the interlocked configuration, the stabilization fins 957 and the perimeter extensions 942 are all radially aligned. The base 944 of the tapered body 941 serves as a flange or stop to arrest axial movement of the outer bumper 911 with respect to the inner wedge 912 in a first direction. The interior face 963 of the stop portion 962 serves as a flange or stop to arrest axial movement of the outer bumper 911 with respect to the inner wedge 912 in a second direction opposite the first direction.

The outer bumper 911 is configured to expand, i.e., elastically deform, around the inner wedge 912 and to be disposed on the seating hub 952 of the inner wedge 912 when the outer bumper 911 and the inner wedge 912 are in an interlocked configuration. As discussed above, the seating hub 952 of the inner wedge 912 has an outer diameter greater than that of the inner wall 954 such that, when the outer bumper 911 is disposed on the seating hub 952, the outer bumper 911 remains in an expanded configuration. The expanded configuration of the outer bumper 911 causes an increase in its effective diameter.

When disposed over the seating hub 952, the valve stops 931 of the outer bumper 911 have an increased effective diameter due to expansion caused by the outer diameter of the seating hub 952. In the interlocked configuration of the multipart retention bumper 901, the abutment surface 971 of the valve stop 931 of the outer bumper 911 may be approximately flush with the exterior face 961 of the stop portion 962 of the inner wedge 912. Approximately flush, as used herein, refers to the abutment surface 971 and the exterior face 961 being coplanar to within 2mm, within 1mm, within 0.5 mm, and/or within 0.1 mm. This arrangement creates an approximately continuous face (within the above tolerances) at the increased effective diameter, i.e., the second radially expanded size, for the retention bumper 901 that faces the prosthetic valve on a balloon catheter. This continuous face serves to prevent axial migration of the prosthetic valve during a valve delivery operation. The continuous face created by the exterior face 961 and the abutment surface 971 is substantially perpendicular to a central axis of the retention bumper 901.

In embodiments, each of the inner wedges 912 and outer bumpers 911 are configured with outer diameters between approximately 0.1 inches and 0.25 inches, between 0.15 and 0.22 inches, between 0.19 and 0.21 inches, or approximately 0.2 inches in their natural resting states. Such sizes correspond to a first radially unexpanded size and may be suitable for entry into the opening of a balloon of a balloon catheter. In embodiments, the multipart retention bumpers 901 may have a maximum outer diameter between 0.25 inches and 0.35 inches, between 0.27 and 0.33 inches, between 0.29 and 0.31 inches, or approximately 0.3 inches after the inner wedges 912 and the outer bumpers 911 are interlocked. Such sizes correspond to a second radially expanded size and are suitable for prevention of axial migration of a prosthetic heart valve located between the retention bumpers 901 when the prosthetic heart valve is subject to force.

Prior to expansion over the inner wedge 912, the outer bumper 911 has an effective diameter at the first radially unexpanded size smaller than a balloon opening. After expansion, the outer bumper 911 has an effective diameter at the second radially expanded size great enough to retain a prosthetic heart valve. Seating the outer bumper 911 on the seating hub 952 of the inner wedge 912 causes the effective diameter of the outer bumper 911 to expand from the first radially unexpanded size to the second radially expanded size.

When the outer bumper 911 is disposed on the seating hub 952 via expanding over the tapered body 941, gaps are created between the valve stops 931 due to the expansion of the spoked perimeter 930. The gaps, or flow recesses 933, allow inflation fluid to flow between the valve stops 931 (in the flow recesses 933) without significant obstruction.

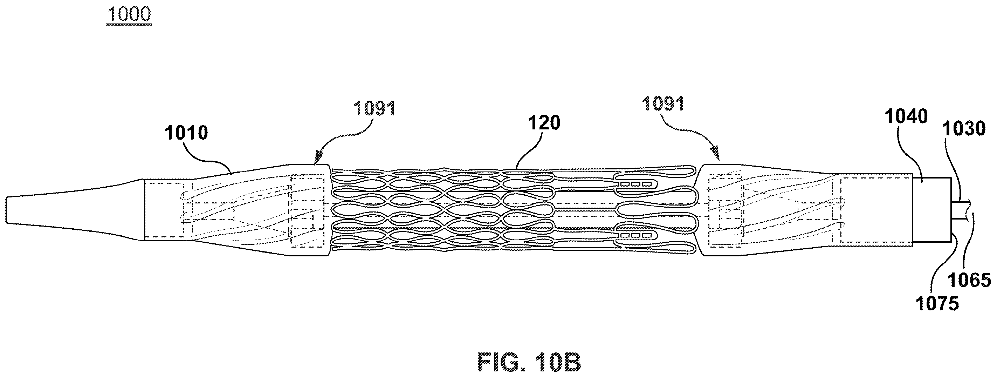

FIGS. 10A and 10B illustrate the retention bumpers 901 included in a balloon catheter 1000. The balloon catheter 1000 includes a distal tip 1060, an inner shaft 1030, an outer shaft 1040, and a balloon 1010 having a balloon opening 1049. The inner shaft 1030 and outer shaft 1040 define an inflation lumen 1075 located therebetween. The balloon catheter 1000 is similar in structure and function to the balloon catheters 600 and 800 but uses the retention bumpers 901. Assembly of the balloon catheter 1000 may be performed similarly to assembly of the balloon catheter 600, as described above, with the outer bumpers 911 being interlocked with the inner wedges 912 by pulling and/or pushing them over the tapered body 941 to be seated on the seating hub 952. In variations of the assembly process, various steps may take place in a different order according to different requirements as described herein.

The prosthetic heart valve 120 is crimped over the pleated and folded balloon 1010, as shown in FIG. 10B. Wrapping the balloon 1010 around the inner shaft 1030 and the retention bumpers 901 creates stops 1091 at the portions where the balloon 1010 wraps over the retention bumpers 901. The stops 1091 are protruding portions of the balloon 1010 that prevent a prosthetic heart valve 120 crimped over the balloon 1010 from migrating during delivery. The prosthetic heart valve 120 is crimped over the balloon 1010 in the space between the stops 1091 created by the multipart retention bumpers 901. Finally, the balloon catheter 1000 is completed via any other processes required, including, for example, attaching the inner shaft and the outer shaft to a proximal handle.

Due to substantial perpendicularity of the continuous face created by the exterior face 961 and the abutment surface 971, the stops 1091 provide an abrupt transition at the ends of the portion of the balloon 1010 where the prosthetic heart valve 120 is crimped. This abrupt transition increases the retention force relative to a sloped or less abrupt transition by preventing the prosthetic heart valve 120 from riding up a sloped transition.

After completion of assembly, the balloon catheter 1000 may be employed to deliver the prosthetic heart valve 120 to a treatment location. The prosthetic heart valve 120 is delivered by the balloon catheter 1000 to a site of deployment. During the delivery process, the balloon catheter 1000 may be guided by a guidewire passing through a guidewire lumen 1065. During delivery of the prosthetic heart valve 120, the multipart retention bumpers 901 serve to maintain the axial position of the prosthetic heart valve 120 and reduce or prevent axial migration of the prosthetic heart valve 120. The stops 1091 provide axial force or pressure to the prosthetic heart valve 120 to maintain the position of the prosthetic heart valve 120 when it is subject to axial or other forces during delivery, for example, by contact with an introducer sheath. When axial force is applied to the stop 1091 (and therefore to the outer bumpers 911), the interior face 963 of the stop portion 962 and the base 944 of the tapered body 941 press against the inner walls 954 and the outer walls 953 of the spoked perimeter 930 to maintain the axial position of each outer bumper 911.

Once delivered, the prosthetic heart valve 120 is deployed through operation of the balloon catheter 1000. Inflation fluid, e.g., saline, contrast fluid, etc., is delivered to the balloon 1010 via the inflation lumen 1075 disposed between the inner shaft 1030 and the outer shaft 1040. The inflation fluid travels through the flow recesses 933 of the outer bumpers 911. Thus, the star shape of the retention bumpers 901 provides fluid pathways to facilitate inflation of the balloon 1010. The path of the inflation fluid is shown by arrows 1099 in FIG. 10A.

Due to the lack of obstruction in the fluid flow pathways, inflation fluid is permitted to flow past the retention bumpers 901 along substantially longitudinal streamlines. Thus, the retention bumpers 901 reduce, minimize, eliminate, or otherwise limit radial flow of inflation fluid. As used herein, substantially longitudinal streamlines refer to fluid flows having streamlines approximately parallel to a designated axis. For example, a substantially longitudinal flow in a balloon catheter may have streamlines approximately parallel to a central axis of the balloon catheter. Approximately parallel may include streamlines within 10 degrees, within 5 degrees, and/or within 1 degree of parallel. Longitudinal flows represent fluid flows along the length of the balloon catheter. Radial flows refer to fluid flows having streamlines that deviate from parallelism with a designated axis, having a significant orthogonal vector component. A streamline with a significant orthogonal vector component may deviate from parallel by 10 degrees or more. Radial flows represent flows towards or away from the axial center of the balloon catheter.

FIGS. 11A and 11B illustrate examples of tools configured for facilitating the assembly of the inner bumpers and outer bumpers, described herein. FIG. 11A illustrates a double pronged bumper assembly tool 1100. The double pronged assembly tool 1100 includes two shafts 1101 having hooks 1102 disposed on one end thereof. At the end of the shafts 1101 opposite the hooks 1102, the shafts 1101 are joined at the hinge 1103. Each shaft 1101 is an elongated structure such as a tube or mandrel and may have a cylindrical, square, or other profile. The hooks 1102 project from the shafts 1101 at an angle configured to facilitate grasping or pulling of the outer bumpers 511, 711, 911. In embodiments, the hooks 1102 may extend from the shafts 1101 at an angle between 30° and 120°. The hinge 1103 may be a living hinge and/or a mechanical hinge. The double pronged assembly tool 1100 is adapted in size to be inserted into the neck or opening of a balloon. After insertion into the balloon, the hooks 1102 may be used to grasp or catch the outer bumpers 511, 711, 911 and provide the pulling force necessary to pull the outer bumpers 511, 711 over the inner wedges 512, 712, 912. Altematively, the double pronged assembly tool 1100 may be employed to push the outer bumpers 511, 711, 911 over the inner wedges 512, 712. FIG. 11B illustrates a singled pronged bumper assembly tool 1110. The single pronged assembly tool 1110 includes a shaft 1111 having a hook 1112 disposed on one end thereof. The shaft 1111 is an elongated structure such as a tube or mandrel and may have a cylindrical, square, or other profile. The hook 1112 projects from the shafts 1111 at an angle configured to facilitate grasping or pulling of the outer bumpers 511, 711, 911. In embodiments, the hook 1112 may extend from the shaft 1111 at an angle between 30° and 120°. The single pronged assembly tool 1110 is adapted in size to be inserted into the neck or opening of a balloon. After insertion into the balloon, the hook 1112 may be used to grasp or catch the outer bumpers 511, 711, 911 and provide the pulling force necessary to pull the outer bumpers 511, 711, 911 over the inner wedges 512, 712, 912. Alternatively, the single pronged assembly tool 1110 may be employed to push the outer bumpers 511, 711, 911 over the inner wedges 512, 712, 912. In embodiments, multiple single pronged assembly tools 1110 may be used to facilitate bumper assembly. In further embodiments, any combination of multiple single pronged assembly tools 1110 and/or multiple double pronged assembly tools 1100 may be employed to facilitate bumper assembly.

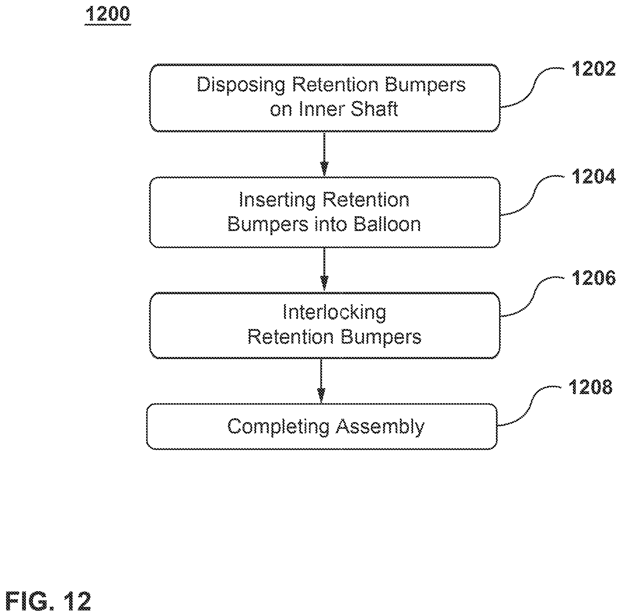

FIG. 12 is a flow chart of a balloon catheter assembly process 1200 for assembly of a balloon catheter involving multipart retention bumpers. The devices and structures described herein reduce or prevent prosthetic heart valve migration during valve delivery. Methods of assembling a balloon catheter described below are consistent with any multipart retention bumpers described herein. In particular, the process 1200 is compatible with embodiments that employ expandable retention bumpers, for example, those embodiments associated with FIGS. 5A-8H, and with any combination of the embodiments described herein. It is not necessary that the following operations of process 1200 occur in the order in which they are described.

In an operation 1202 of balloon catheter assembly process 1200, at least some of the multipart retention bumpers are disposed on the inner shaft of a balloon catheter to form a partial assembly. The inner wedge portions of the multipart retention bumpers are secured to the inner shaft, e.g., via over molding. One or more outer bumper portions of the multipart retention bumpers may be disposed over the shaft. The one or more outer bumper portions are not secured and are permitted freely slide on the inner shaft. In embodiments, a distal tip may also be secured to the partial assembly at this time. In some embodiments, the distal outer bumper is disposed on the inner shaft at a location distal of the distal inner wedge and the proximal outer bumper is disposed on the inner shaft at a location proximal of the proximal inner wedge. In embodiments, the proximal inner wedge is not disposed on the inner shaft at this stage. In some embodiments, both outer bumpers are disposed on the shaft between the inner wedges.

In an operation 1204, the distal and proximal multipart retention bumpers, including the inner wedges and the outer bumpers are inserted into a balloon of the balloon catheter. Prior to insertion, the balloon is bonded to the outer shaft of the balloon catheter. During insertion, the distal and proximal multipart retention bumpers, e.g., the distal and proximal inner wedges and the distal and proximal outer bumpers, are maintained at a first radially unexpanded size. In embodiments, maintaining the retention bumpers at a first radially unexpanded size may involve maintaining the inner wedges and outer bumpers in a non-interlocked configuration. In further embodiments, maintaining the multipart retention bumpers at a first radially unexpanded size may involve maintaining the inner wedges and outer bumpers in a partially-interlocked configuration that does not result in expansion of the outer bumper. In embodiments, the balloon is bonded, e.g., via heat bonding, to the distal tip of the catheter at this stage.

In an operation 1206, the distal and proximal multipart retention bumpers are interlocked and expanded from the first radially unexpanded size to the second radially expanded size. The free moving outer bumpers are stretched over the secured inner wedges and located on the seating hubs thereof. In embodiments, the outer bumpers may be stretched over the secured inner wedges from either end of the inner wedges. The inner wedges and the outer bumpers may be secured to one another by the mating of features of the elastic outer bumper and the rigid inner wedge, as described herein. To cause interlock, a tool may be inserted into the opening of the balloon of the balloon catheter to press the outer bumper over the inner wedge. In some embodiments, the inner wedge and the outer bumper may be manually manipulated through the material of the balloon.

In some embodiments, the distal multipart retention bumper may be interlocked prior to inserting the proximal multipart retention bumper into the balloon.

In an operation 1208, assembly of the balloon catheter is completed. Completing the balloon catheter assembly includes the completion of all further required steps. For example, the outer shaft may be assembled to the catheter at this time by inserting the inner shaft into the outer shaft and bonding the outer shaft to the balloon. In some embodiments, if the balloon has not yet been bonded to the distal tip, this operation may be completed at this stage. Completing balloon catheter assembly may further involve steps such as folding the balloon, attaching the inner shaft and the outer shaft to a proximal handle, crimping a prosthetic heart valve over the balloon, and any other step required to complete assembly of the balloon catheter.

As discussed above, e.g., with respect to FIGS. 8A-8H, the assembly process 1200 may include several variations without departing from the scope of this disclosure. For example, different portions of the multipart retention bumpers may be secured prior to assembly. In further embodiments, expansion of the multipart retention bumpers inside the balloon may be completed prior to or subsequent to securing the distal tip to the inner shaft, securing the outer shaft to the balloon, and/or securing the balloon to the distal tip. Suitable ordering of these processes may be selected to accommodate the expansion of the multipart retention bumpers.

The foregoing description has been presented for purposes of illustration and enablement and is not intended to be exhaustive or to limit the invention to the precise form disclosed. Other modifications and variations are possible in light of the above teachings. The embodiments and examples were chosen and described in order to best explain the principles of the invention and its practical application and to thereby enable others skilled in the art to best utilize the invention in various embodiments and various modifications as are suited to the particular use contemplated.

## Claims

1. A balloon catheter (100, 600, 800, 1000) for deploying a prosthetic heart valve (120) through balloon inflation, comprising:
an inner shaft (130, 630, 830, 1030) defining a guidewire lumen (165, 665, 865, 1065);
an outer shaft (140, 640, 840, 1040) surrounding the inner shaft defining an inflation lumen (170, 675, 875, 1075) between the outer shaft and the inner shaft;
a distal portion at a distal end of the inner shaft;
a balloon (110, 610, 810, 1010) disposed at the distal portion such that fluid delivered to the balloon via the inflation lumen causes the balloon to inflate;
a multipart retention bumper (501, 701, 901) having a star shape, the multipart retention bumper being secured to the inner shaft inside the balloon and configurable in an interlocked configuration and a non-interlocked configuration, the multipart retention bumper including an inner wedge (512, 712, 912) and an outer bumper (511, 711, 911), wherein the outer bumper is star shaped.

2. The balloon catheter of claim 1, wherein the multipart retention bumper is a proximal multipart retention bumper, the inner wedge is a proximal inner wedge (512B, 712B, 912), and the outer bumper is a proximal outer bumper (511B, 711B, 911), and the balloon catheter further comprises:
a distal multipart retention bumper having a star shape, the distal multipart retention bumper being secured to the inner shaft inside the balloon and configurable in an interlocked configuration and a non-interlocked configuration, the distal multipart retention bumper including a distal inner wedge (512A, 712A, 912) and a distal outer bumper (511A, 711A, 911).

3. The balloon catheter of claim 2, wherein in the non-interlocked configuration, the proximal inner wedge and the proximal outer bumper of the proximal multipart retention bumper each have a radially unexpanded size adapted to fit within a neck of the balloon during an assembly process, and
wherein in the non-interlocked configuration, the distal inner wedge and the distal outer bumper of the distal multipart retention bumper have radially unexpanded sizes adapted to fit within the neck of the balloon during the assembly process; or wherein in the interlocked configuration the proximal inner wedge and the proximal outer bumper of the proximal multipart retention bumper have a radially expanded size adapted to maintain an axial position of the prosthetic heart valve when the prosthetic heart valve is subject to axial force, and
wherein in the interlocked configuration the distal inner wedge and the distal outer bumper of the distal multipart retention bumper have a radially expanded size adapted to maintain the axial position of the prosthetic heart valve when the prosthetic heart valve is subject to axial force; or wherein the proximal inner wedge is configured to expand a diameter of the proximal outer bumper to a radially expanded size when the proximal inner wedge and the proximal outer bumper are in the interlocked configuration, and
wherein the distal inner wedge is configured to expand a diameter of the distal outer bumper to the radially expanded size when the distal inner wedge and the distal outer bumper are in the interlocked configuration.

4. The balloon catheter of claim 2, wherein the proximal outer bumper is configured to stretch over the proximal inner wedge to achieve the interlocked configuration, and
wherein the distal outer bumper is configured to stretch over the distal inner wedge to achieve the interlocked configuration; or wherein the proximal inner wedge includes a seating hub (752, 952) having one or more stabilization fins (757, 957) and the star shape of the proximal outer bumper includes a spoked perimeter (730, 930) having one or more spokes (731), optionally forming a plurality of valve stops (931).

5. The balloon catheter of claim 2, wherein in the interlocked configuration an exterior face of a stop portion of the proximal inner wedge is approximately flush with an abutment surface of the proximal outer bumper; and
wherein in the interlocked configuration an exterior face of a stop portion of the distal inner wedge is approximately flush with an abutment surface of the distal outer bumper; and optionally wherein the proximal multipart retention bumper further includes flow recesses (933) between the plurality of valve stops (931), the flow recesses being configured to permit longitudinal inflation fluid flow and limit radial inflation fluid flow.

6. The balloon catheter of claim 2, wherein the proximal inner wedge and the distal inner wedge are substantially non-compressible during steps of manufacture, assembly, and use of the balloon catheter, and
wherein the proximal outer bumper and the distal outer bumper are expandable; or wherein the proximal inner wedge and the distal inner wedge are over molded to the inner shaft; or wherein the proximal multipart retention bumper and the distal multipart retention bumper are secured to the inner shaft with a space therebetween to accommodate the prosthetic heart valve.

7. The balloon catheter of claim 2, wherein the proximal inner wedge has a star shape and includes a ridged tapered body (541, 741) having one or more extension arms (542, 742) and the star shape of the proximal outer bumper includes a spoked perimeter (530, 730) having one or more spokes (531, 731).

8. The balloon catheter of claim 7, wherein, in the interlocked configuration, the one or more extension arms are radially aligned with the one or more spokes; and optionally wherein the proximal multipart retention bumper further includes flow recesses (533, 733) between one or more extension arms radially aligned with the one or more spokes, the flow recesses being configured to permit longitudinal inflation fluid flow and limit radial inflation fluid flow.

9. A method of assembling a balloon catheter (100, 600, 800, 1000) adapted for deploying a prosthetic heart valve (120) through balloon inflation, the method comprising:
disposing an outer bumper (511, 711, 911) over an inner shaft (130, 630, 830, 1030) of the balloon catheter at a distal portion of the inner shaft wherein the outer bumper is star shaped;
securing an inner wedge (512, 712, 912) to an inner shaft of the balloon catheter at the distal portion of the inner shaft;
positioning a balloon (110, 610, 810, 1010) of the balloon catheter over the outer bumper and the inner wedge by passing the outer bumper and the inner wedge into the balloon via an opening of the balloon; and
interlocking the inner wedge and the outer bumper to form a multipart retention bumper.

10. The method of assembling the balloon catheter of claim 9,
wherein the multipart retention bumper is a proximal multipart retention bumper, the inner wedge is a proximal inner wedge, and the outer bumper is a proximal outer bumper, and the method further comprises:
disposing a distal outer bumper over an inner shaft of the balloon catheter at a distal portion of the inner shaft wherein the distal outer bumper is star shaped;
securing a distal inner wedge to an inner shaft of the balloon catheter at the distal portion of the inner shaft; and
positioning a balloon of the balloon catheter over the distal outer bumper and the distal inner wedge by passing the distal outer bumper and the distal inner wedge into the balloon via a distal opening (449, 649, 849, 1049) of the balloon; and
interlocking the distal inner wedge and the distal outer bumper to form a distal multipart retention bumper (501A, 701, 901).

11. The method of assembling the balloon catheter of claim 10,
wherein the proximal inner wedge, the proximal outer bumper, the distal inner wedge, and the distal outer bumper each have a radially unexpanded size,
wherein positioning the balloon over the proximal outer bumper, the distal outer bumper, the proximal inner wedge, and the distal inner wedge is performed without compression of the proximal outer bumper, the distal outer bumper, the proximal inner wedge, and the distal inner wedge; or
wherein the proximal multipart retention bumper and the distal multipart retention bumper each have a radially expanded size adapted to prevent axial migration of the prosthetic heart valve when the prosthetic heart valve is subject to axial force.

12. The method of assembling the balloon catheter of claim 10, further comprising:
crimping the prosthetic heart valve over the balloon between the proximal multipart retention bumper and the distal multipart retention bumper; or wherein securing the proximal inner wedge and the distal inner wedge to the inner shaft includes over molding the proximal inner wedge and the distal inner wedge to the inner shaft.

13. The method of assembling the balloon catheter of claim 10, wherein interlocking the proximal inner wedge and the proximal outer bumper includes aligning an exterior face of a stop portion of the proximal inner wedge to be approximately flush with an abutment surface of the proximal outer bumper stretching the proximal outer bumper over the proximal inner wedge, and
wherein interlocking the proximal inner wedge and the proximal outer bumper includes aligning an exterior face of a stop portion of the distal inner wedge to be approximately flush with an abutment surface of the distal outer bumper; or wherein the proximal inner wedge and the distal inner wedge each include tapered sides,
interlocking the proximal inner wedge and the proximal outer bumper includes expanding the proximal outer bumper by the tapered sides of the proximal inner wedge; and
interlocking the distal inner wedge and the distal outer bumper includes expanding the distal outer bumper by the tapered sides of the distal inner wedge.

14. The method of assembling the balloon catheter of claim 10, further comprising:
aligning stabilization fins (757, 957) of a seating hub (752, 952) of the proximal inner wedge with spokes of a spoked perimeter of the proximal outer bumper prior during interlocking the proximal inner wedge and the proximal outer bumper; and
aligning stabilization fins of a seating hub of the distal inner wedge with spokes of a spoked perimeter of the distal outer bumper prior during interlocking the distal inner wedge and the distal outer bumper; and optionally further comprising establishing, along the proximal multipart retention bumper, flow recesses (733, 933) to provide a longitudinal flow pathway for inflation fluid and limit radial flow of inflation fluid.

15. The method of assembling the balloon catheter of claim 10, further comprising:
aligning extension arms of a ridged tapered body (541, 741) of the proximal inner wedge with spokes (531, 731) of a spoked perimeter (530, 730) of the proximal outer bumper prior during interlocking the proximal inner wedge and the proximal outer bumper; and
aligning extension arms of a ridged tapered body of the distal inner wedge with spokes of a spoked perimeter of the distal outer bumper prior during interlocking the distal inner wedge and the distal outer bumper.

## Patentansprüche

1. Ballonkatheter (100, 600, 800, 1000) zum Anwenden einer Herzklappenprothese (120) durch Ballonaufblasen, umfassend:
einen inneren Schaft (130, 630, 830, 1030), der ein Führungsdrahtlumen (165, 665, 865, 1065) definiert;
einen äußeren Schaft (140, 640, 840, 1040), der den äußeren Schaft umgibt und ein Aufblaslumen (170, 675, 875, 1075) zwischen dem äußeren Schaft und dem inneren Schaft definiert;
einen distalen Abschnitt an einem distalen Ende des inneren Schafts;
einen Ballon (110, 610, 810, 1010), der an dem distalen Abschnitt angeordnet ist, so dass über das Aufblaslumen an den Ballon abgegebenes Fluid bewirkt, dass sich der Ballon aufbläst;
einen mehrteiligen Rückhaltepuffer (501, 701, 901) mit einer Sternform, wobei der mehrteilige Rückhaltepuffer an dem inneren Schaft innerhalb des Ballons befestigt ist und in einer verriegelten Konfiguration und einer nicht verriegelten Konfiguration konfigurierbar ist, wobei der mehrteilige Rückhaltepuffer einen inneren Keil (512, 712, 912) und einen äußeren Puffer (511, 711, 911) einschließt, wobei der äußere Puffer sternförmig ist.

2. Ballonkatheter nach Anspruch 1, wobei der mehrteilige Rückhaltepuffer ein proximaler mehrteiliger Rückhaltepuffer ist, der innere Keil ein proximaler innerer Keil (512B, 712B, 912) ist und der äußere Puffer ein proximaler äußerer Puffer (511B, 711B, 911) ist und der Ballonkatheter ferner umfasst:
einen distalen mehrteiligen Rückhaltepuffer mit einer Sternform, wobei der distale mehrteilige Rückhaltepuffer an dem inneren Schaft innerhalb des Ballons befestigt ist und in einer verriegelten Konfiguration und einer nicht verriegelten Konfiguration konfigurierbar ist, wobei der distale mehrteilige Rückhaltepuffer einen distalen inneren Keil (512A, 712A, 912) und einen distalen äußeren Puffer (511A, 711A, 911) einschließt.

3. Ballonkatheter nach Anspruch 2, wobei in der nicht verriegelten Konfiguration der proximale innere Keil und der proximale äußere Puffer des proximalen mehrteiligen Rückhaltepuffers jeweils eine radial nicht expandierte Größe aufweisen, die ausgelegt ist, um während eines Montageprozesses in einen Hals des Ballons zu passen, und
wobei in der nicht verriegelten Konfiguration der distale innere Keil und der distale äußere Puffer des distalen mehrteiligen Rückhaltepuffers radial nicht expandierte Größen aufweisen, die ausgelegt sind, um während des Montageprozesses in den Hals des Ballons zu passen; oder wobei in der verriegelten Konfiguration der proximale innere Keil und der proximale äußere Puffer des proximalen mehrteiligen Rückhaltepuffers eine radial erweiterte Größe aufweisen, die ausgelegt ist, um eine axiale Position der Herzklappenprothese aufrechtzuerhalten, wenn die Herzklappenprothese einer axialen Kraft ausgesetzt ist, und
wobei in der verriegelten Konfiguration der distale innere Keil und der distale äußere Puffer des distalen mehrteiligen Rückhaltepuffers eine radial erweiterte Größe aufweisen, die ausgelegt ist, um die axiale Position der Herzklappenprothese aufrechtzuerhalten, wenn die Herzklappenprothese einer axialen Kraft ausgesetzt ist; oder wobei der proximale innere Keil konfiguriert ist, um einen Durchmesser des proximalen äußeren Puffers auf eine radial expandierte Größe zu expandieren, wenn sich der proximale innere Keil und der proximale äußere Puffer in der verriegelten Konfiguration befinden, und
wobei der distale innere Keil konfiguriert ist, um einen Durchmesser des distalen äußeren Puffers auf die radial expandierte Größe zu expandieren, wenn sich der distale innere Keil und der distale äußere Puffer in der verriegelten Konfiguration befinden.

4. Ballonkatheter nach Anspruch 2, wobei der proximale äußere Puffer konfiguriert ist, um sich über den proximalen inneren Keil zu dehnen, um die verriegelte Konfiguration zu erreichen, und
wobei der distale äußere Puffer konfiguriert ist, um sich über den distalen inneren Keil zu dehnen, um die verriegelte Konfiguration zu erreichen; oder wobei der proximale innere Keil eine Sitznabe (752, 952) mit einer oder mehreren Stabilisierungsrippen (757, 957) einschließt und die Sternform des proximalen äußeren Puffers einen speichenförmigen Umfang (730, 930) mit einer oder mehreren Speichen (731) einschließt, die optional eine Vielzahl von Ventilanschlägen (931) bilden.

5. Ballonkatheter nach Anspruch 2, wobei in der verriegelten Konfiguration eine Außenfläche eines Anschlagabschnitts des proximalen inneren Keils etwa bündig mit einer Widerlageroberfläche des proximalen äußeren Puffers ist; und
wobei in der verriegelten Konfiguration eine Außenfläche eines Anschlagabschnitts des distalen inneren Keils etwa bündig mit einer Widerlageroberfläche des distalen äußeren Puffers ist; und optional wobei der proximale mehrteilige Rückhaltepuffer ferner Strömungsvertiefungen (933) zwischen der Vielzahl von Ventilanschlägen (931) einschließt, wobei die Strömungsvertiefungen konfiguriert sind, um longitudinale Aufblasfluidströmung zu ermöglichen und radiale Aufblasfluidströmung zu begrenzen.

6. Ballonkatheter nach Anspruch 2, wobei der proximale innere Keil und der distale innere Keil während der Schritte der Herstellung, Montage und Verwendung des Ballonkatheters im Wesentlichen nicht komprimierbar sind und
wobei der proximale äußere Puffer und der distale äußere Puffer expandierbar sind; oder wobei der proximale innere Keil und der distale innere Keil über den inneren Schaft umspritzt sind; oder wobei der proximale mehrteilige Rückhaltepuffer und der distale mehrteilige Rückhaltepuffer an dem inneren Schaft mit einem Zwischenraum dazwischen befestigt sind, um die Herzklappenprothese aufzunehmen.

7. Ballonkatheter nach Anspruch 2, wobei der proximale innere Keil eine Sternform aufweist und einen gerippten, sich verjüngenden Körper (541, 741) mit einem oder mehreren Verlängerungsarmen (542, 742) einschließt und die Sternform des proximalen äußeren Puffers einen speichenförmigen Umfang (530, 730) mit einer oder mehreren Speichen (531, 731) einschließt.

8. Ballonkatheter nach Anspruch 7, wobei in der verriegelten Konfiguration der eine oder die mehreren Verlängerungsarme radial mit der einen oder den mehreren Speichen ausgerichtet sind; und optional wobei der proximale mehrteilige Rückhaltepuffer ferner Strömungsvertiefungen (533, 733) zwischen einem oder mehreren Verlängerungsarmen einschließt, die radial mit der einen oder den mehreren Speichen ausgerichtet sind, wobei die Strömungsvertiefungen konfiguriert sind, um longitudinale Aufblasfluidströmung zu ermöglichen und radiale Aufblasfluidströmung zu begrenzen.

9. Verfahren zum Montieren eines Ballonkatheters (100, 600, 800, 1000), der zum Anwenden einer Herzklappenprothese (120) durch Ballonaufblasen ausgelegt ist, wobei das Verfahren umfasst:
Anordnen eines äußeren Puffers (511, 711, 911) über einem inneren Schaft (130, 630, 830, 1030) des Ballonkatheters an einem distalen Abschnitt des inneren Schafts, wobei der äußere Puffer sternförmig ist;
Befestigen eines inneren Keils (512, 712, 912) an einem inneren Schaft des Ballonkatheters am distalen Abschnitt des inneren Schafts;
Positionieren eines Ballons (110, 610, 810, 1010) des Ballonkatheters über dem äußeren Puffer und dem inneren Keil durch Einführen des äußeren Puffers und des inneren Keils in den Ballon über eine Öffnung des Ballons; und
Verriegeln des inneren Keils und des äußeren Puffers zum Bilden eines mehrteiligen Rückhaltepuffers.

10. Verfahren zum Montieren des Ballonkatheters nach Anspruch 9,
wobei der mehrteilige Rückhaltepuffer ein proximaler mehrteiliger Rückhaltepuffer ist, der innere Keil ein proximaler innerer Keil ist und der äußere Puffer ein proximaler äußerer Puffer ist und das Verfahren ferner umfasst:
Anordnen eines distalen äußeren Puffers über einem inneren Schaft des Ballonkatheters an einem distalen Abschnitt des inneren Schafts, wobei der distale äußere Puffer sternförmig ist;
Befestigen eines distalen inneren Keils an einem inneren Schaft des Ballonkatheters am distalen Abschnitt des inneren Schafts; und
Positionieren eines Ballons des Ballonkatheters über dem distalen äußeren Puffer und dem distalen inneren Keil durch Einführen des distalen äußeren Puffers und des distalen inneren Keils in den Ballon über eine distale Öffnung (449, 649, 849, 1049) des Ballons; und
Verriegeln des distalen inneren Keils und des distalen äußeren Puffers zum Bilden eines distalen mehrteiligen Rückhaltepuffers (501A, 701, 901).

11. Verfahren zum Montieren des Ballonkatheters nach Anspruch 10,
wobei der proximale innere Keil, der proximale äußere Puffer, der distale innere Keil und der distale äußere Puffer jeweils eine radial nicht expandierte Größe aufweisen,
wobei das Positionieren des Ballons über dem proximalen äußeren Puffer, dem distalen äußeren Puffer, dem proximalen inneren Keil und dem distalen inneren Keil ohne Kompression des proximalen äußeren Puffers, des distalen äußeren Puffers, des proximalen inneren Keils und des distalen inneren Keils durchgeführt wird; oder
wobei der proximale mehrteilige Rückhaltepuffer und der distale mehrteilige Rückhaltepuffer jeweils eine radial erweiterte Größe aufweisen, die ausgelegt ist, um eine axiale Migration der Herzklappenprothese zu verhindern, wenn die Herzklappenprothese einer axialen Kraft ausgesetzt ist.

12. Verfahren zum Montieren des Ballonkatheters nach Anspruch 10, ferner umfassend:
Crimpen der Herzklappenprothese über dem Ballon zwischen dem proximalen mehrteiligen Rückhaltepuffer und dem distalen mehrteiligen Rückhaltepuffer; oder wobei das Befestigen des proximalen inneren Keils und des distalen inneren Keils an dem inneren Schaft das Umspritzen des proximalen inneren Keils und des distalen inneren Keils an dem inneren Schaft einschließt.

13. Verfahren zum Montieren des Ballonkatheters nach Anspruch 10, wobei das Verriegeln des proximalen inneren Keils und des proximalen äußeren Puffers das Ausrichten einer Außenfläche eines Anschlagabschnitts des proximalen inneren Keils, um etwa bündig mit einer Widerlageroberfläche des proximalen äußeren Puffers zu sein, das Dehnen des proximalen äußeren Puffers über den proximalen inneren Keil einschließt, und
wobei das Verriegeln des proximalen inneren Keils und des proximalen äußeren Puffers das Ausrichten einer Außenfläche eines Anschlagabschnitts des distalen inneren Keils einschließt, um etwa bündig mit einer Widerlageroberfläche des distalen äußeren Puffers zu sein; oder wobei der proximale innere Keil und der distale innere Keil jeweils sich verjüngende Seiten einschließen,
das Verriegeln des proximalen inneren Keils und des proximalen äußeren Puffers das Expandieren des proximalen äußeren Puffers durch die sich verjüngenden Seiten des proximalen inneren Keils einschließt; und
das Verriegeln des distalen inneren Keils und des distalen äußeren Puffers das Expandieren des distalen äußeren Puffers durch die sich verjüngenden Seiten des distalen inneren Keils einschließt.

14. Verfahren zum Montieren des Ballonkatheters nach Anspruch 10, ferner umfassend:
Ausrichten von Stabilisierungsrippen (757, 957) einer Sitznabe (752, 952) des proximalen inneren Keils mit Speichen eines Speichenumfangs des proximalen äußeren Puffers während des Verriegelns des proximalen inneren Keils und des proximalen äußeren Puffers; und
Ausrichten von Stabilisierungsrippen einer Sitznabe des distalen inneren Keils mit Speichen eines Speichenumfangs des distalen äußeren Puffers während des Verriegelns des distalen inneren Keils und des distalen äußeren Puffers; und optional ferner umfassend das Errichten von Strömungsvertiefungen (733, 933) entlang des proximalen mehrteiligen Rückhaltepuffers, um einen longitudinalen Strömungspfad für Aufblasfluid bereitzustellen und radiale Strömung von Aufblasfluid zu begrenzen.

15. Verfahren zum Montieren des Ballonkatheters nach Anspruch 10, ferner umfassend:
Ausrichten von Verlängerungsarmen eines gerippten, sich verjüngenden Körpers (541, 741) des proximalen inneren Keils mit Speichen (531, 731) eines Speichenumfangs (530, 730) des proximalen äußeren Puffers während des Verriegelns des proximalen inneren Keils und des proximalen äußeren Puffers; und
Ausrichten von Verlängerungsarmen eines gerippten, sich verjüngenden Körpers des distalen inneren Keils mit Speichen eines Speichenumfangs des distalen äußeren Puffers während des Verriegelns des distalen inneren Keils und des distalen äußeren Puffers.

## Revendications

1. Cathéter à ballonnet (100, 600, 800, 1000) permettant de déployer une valvule cardiaque prothétique (120) par gonflage par ballonnet, comprenant :
une tige intérieure (130, 630, 830, 1030) définissant une lumière de fil-guide (165, 665, 865, 1065) ;
une tige extérieure (140, 640, 840, 1040) entourant la tige intérieure définissant une lumière de gonflage (170, 675, 875, 1075) entre la tige extérieure et la tige intérieure ;
une partie distale au niveau d'une extrémité distale de la tige intérieure ;
un ballonnet (110, 610, 810, 1010) disposé au niveau de la partie distale de telle sorte que le fluide délivré au ballonnet par l'intermédiaire de la lumière de gonflage provoque le gonflage du ballonnet ;
un butoir de rétention en plusieurs parties (501, 701, 901) ayant une forme d'étoile, le butoir de rétention en plusieurs parties étant fixé à la tige intérieure à l'intérieur du ballonnet et pouvant être configuré dans une configuration verrouillée et une configuration non verrouillée, le butoir de rétention en plusieurs parties comportant une cale interne (512, 712, 912) et un butoir externe (511,711,911), dans lequel le butoir externe est en forme d'étoile.

2. Cathéter à ballonnet selon la revendication 1, dans lequel le butoir de rétention en plusieurs parties est un butoir de rétention en plusieurs parties proximal, la cale interne est une cale interne proximale (512B, 712B, 912), et le butoir externe est un butoir externe proximal (511B, 711B, 911), et le cathéter à ballonnet comprend en outre :
un butoir de rétention en plusieurs parties distal ayant une forme d'étoile, le butoir de rétention en plusieurs parties distal étant fixé à la tige intérieure à l'intérieur du ballonnet et pouvant être configuré dans une configuration verrouillée et une configuration non verrouillée, le butoir de rétention en plusieurs parties distal comportant une cale interne distale (512A, 712A, 912) et un butoir externe distal (511A, 711A, 911).

3. Cathéter à ballonnet selon la revendication 2, dans lequel, dans la configuration non verrouillée, la cale interne proximale et le butoir externe proximal du butoir de rétention en plusieurs parties proximal ont chacun une taille radialement non expansée adaptée pour s'ajuster à l'intérieur d'un col du ballonnet pendant un procédé d'assemblage, et
dans lequel, dans la configuration non verrouillée, la cale interne distale et le butoir externe distal du butoir de rétention en plusieurs parties distal ont des tailles radialement non expansées adaptées pour s'ajuster à l'intérieur du col du ballonnet pendant le procédé d'assemblage ; ou dans lequel, dans la configuration verrouillée, la cale interne proximale et le butoir externe proximal du butoir de rétention en plusieurs parties proximal ont une taille radialement expansée adaptée pour maintenir une position axiale de la valvule cardiaque prothétique lorsque la valvule cardiaque prothétique est soumise à une force axiale, et
dans lequel, dans la configuration verrouillée, la cale interne distale et le butoir externe distal du butoir de rétention en plusieurs parties distal ont une taille radialement expansée adaptée pour maintenir la position axiale de la valvule cardiaque prothétique lorsque la valvule cardiaque prothétique est soumise à une force axiale ; ou dans lequel la cale interne proximale est conçue pour s'expanser d'un diamètre du butoir externe proximal à une taille radialement expansée lorsque la cale interne proximale et le butoir externe proximal sont dans la configuration verrouillée, et
dans lequel la cale interne distale est conçue pour expanser un diamètre du butoir externe distal jusqu'à la taille radialement expansée lorsque la cale interne distale et le butoir externe distal sont dans la configuration verrouillée.

4. Cathéter à ballonnet selon la revendication 2, dans lequel le butoir externe proximal est conçu pour s'étirer sur la cale interne proximale afin d'obtenir la configuration verrouillée, et
dans lequel le butoir externe distal est conçu pour s'étirer sur la cale interne distale afin d'obtenir la configuration verrouillée ; ou dans lequel la cale interne proximale comporte un moyeu d'assise (752, 952) ayant une ou plusieurs ailettes de stabilisation (757, 957) et la forme d'étoile du butoir externe proximal comporte un périmètre à rayons (730, 930) ayant un ou plusieurs rayons (731), formant facultativement une pluralité de butées de valvule (931).

5. Cathéter à ballonnet selon la revendication 2, dans lequel, dans la configuration verrouillée, une face extérieure d'une partie de butée de la cale interne proximale affleure approximativement une surface de butée du butoir externe proximal ; et
dans lequel, dans la configuration verrouillée, une face extérieure d'une partie de butée de la cale interne distale affleure approximativement une surface de butée du butoir externe distal ; et éventuellement dans lequel le butoir de rétention en plusieurs parties proximal comporte en outre des évidements d'écoulement (933) entre la pluralité de butées de valvule (931), les évidements d'écoulement étant conçus pour permettre un écoulement longitudinal de fluide de gonflage et limiter un écoulement radial de fluide de gonflage.

6. Cathéter à ballonnet selon la revendication 2, dans lequel la cale interne proximale et la cale interne distale sont sensiblement non compressibles pendant les étapes de fabrication, d'assemblage et d'utilisation du cathéter à ballonnet, et
dans lequel le butoir externe proximal et le butoir externe distal sont expansibles ; ou dans lequel la cale interne proximale et la cale interne distale sont surmoulées à la tige intérieure ; ou dans lequel le butoir de rétention en plusieurs parties proximal et le butoir de rétention en plusieurs parties distal sont fixés à la tige intérieure avec un espace entre eux pour accueillir la valvule cardiaque prothétique.

7. Cathéter à ballonnet selon la revendication 2, dans lequel la cale interne proximale a une forme d'étoile et comporte un corps conique effilé (541, 741) ayant un ou plusieurs bras d'extension (542, 742) et la forme d'étoile du butoir externe proximal comporte un périmètre à rayons (530, 730) ayant un ou plusieurs rayons (531, 731).

8. Cathéter à ballonnet selon la revendication 7, dans lequel, dans la configuration verrouillée, le ou les bras d'extension sont alignés radialement avec le ou les rayons ; et éventuellement dans lequel le butoir de rétention en plusieurs parties proximal comporte en outre des évidements d'écoulement (533, 733) entre un ou plusieurs bras d'extension alignés radialement sur l'un ou plusieurs rayons, les évidements d'écoulement étant conçus pour permettre un écoulement longitudinal de fluide de gonflage et limiter un écoulement radial de fluide de gonflage.

9. Procédé d'assemblage d'un cathéter à ballonnet (100, 600, 800, 1000) destiné au déploiement d'une valvule cardiaque prothétique (120) par gonflage par ballonnet, le procédé comprenant :
la disposition d'un butoir externe (511, 711, 911) sur une tige intérieure (130, 630, 830, 1030) du cathéter à ballonnet au niveau d'une partie distale de la tige intérieure, dans lequel le butoir externe est en forme d'étoile ;
la fixation d'une cale interne (512, 712, 912) à une tige intérieure du cathéter à ballonnet au niveau de la partie distale de la tige intérieure ;
le positionnement d'un ballonnet (110, 610, 810, 1010) du cathéter à ballonnet sur le butoir externe et la cale interne en faisant passer le butoir externe et la cale interne dans le ballonnet par l'intermédiaire d'une ouverture du ballonnet ; et
l'emboîtement de la cale interne et du butoir externe pour former un butoir de rétention en plusieurs parties.

10. Procédé d'assemblage du cathéter à ballonnet selon la revendication 9,
dans lequel le butoir de rétention en plusieurs parties est un butoir de rétention en plusieurs parties proximal, la cale interne est une cale interne proximale, et le butoir externe est un butoir externe proximal, et le procédé comprend en outre :
la disposition d'un butoir externe distal sur une tige intérieure du cathéter à ballonnet au niveau d'une partie distale de la tige intérieure, dans lequel le butoir externe distal est en forme d'étoile ;
la fixation d'une cale interne distale à une tige intérieure du cathéter à ballonnet au niveau de la partie distale de la tige intérieure ; et
le positionnement d'un ballonnet du cathéter à ballonnet sur le butoir externe distal et la cale interne distale en faisant passer le butoir externe distal et la cale interne distale dans le ballonnet par l'intermédiaire d'une ouverture distale (449, 649, 849, 1049) du ballonnet ; et
l'emboîtement de la cale interne distale et du butoir externe distal pour former un butoir de rétention en plusieurs parties distal (501A, 701, 901).

11. Procédé d'assemblage du cathéter à ballonnet selon la revendication 10,
dans lequel la cale interne proximale, la cale externe proximale, la cale interne distale et le butoir externe distal ont chacun une taille radialement non expansée,
dans lequel le positionnement du ballonnet sur le butoir externe proximal, le butoir externe distal, la cale interne proximale et la cale interne distale est effectué sans compression du butoir externe proximal, du butoir externe distal, de la cale interne proximale et de la cale interne distale ; ou
dans lequel le butoir de rétention en plusieurs parties proximal et le butoir de rétention en plusieurs parties distal ont chacun une taille radialement expansée adaptée pour empêcher la migration axiale de la valvule cardiaque prothétique lorsque la valvule cardiaque prothétique est soumise à une force axiale.

12. Procédé d'assemblage du cathéter à ballonnet selon la revendication 10, comprenant en outre :
le sertissage de la valvule cardiaque prothétique sur le ballonnet entre le butoir de rétention en plusieurs parties proximal et le butoir de rétention en plusieurs parties distal ; ou dans lequel la fixation de la cale interne proximale et de la cale interne distale à la tige intérieure comporte le surmoulage de la cale interne proximale et de la cale interne distale à la tige intérieure.

13. Procédé d'assemblage du cathéter à ballonnet selon la revendication 10, dans lequel l'emboîtement de la cale interne proximale et du butoir externe proximal comporte l'alignement d'une face extérieure d'une partie de butée de la cale interne proximale pour qu'elle affleure approximativement une surface de butée du butoir externe proximal, étirant le butoir externe proximal sur la cale interne proximale, et
dans lequel l'emboîtement de la cale interne proximale et du butoir externe proximal comporte l'alignement d'une face extérieure d'une partie de butée de la cale interne distale pour qu'elle affleure approximativement une surface de butée du butoir externe distal ; ou dans lequel la cale interne proximale et la cale interne distale comportent chacune des côtés effilés,
l'emboîtement de la cale interne proximale et du butoir externe proximal comporte l'expansion du butoir externe proximal par les côtés effilés de la cale interne proximale ; et
l'emboîtement de la cale interne distale et du butoir externe distal comporte l'expansion du butoir externe distal par les côtés effilés de la cale interne distale.

14. Procédé d'assemblage du cathéter à ballonnet selon la revendication 10, comprenant en outre :
l'alignement d'ailettes de stabilisation (757, 957) d'un moyeu d'assise (752, 952) de la cale interne proximale avec des rayons d'un périmètre à rayons du butoir externe proximal au préalable pendant l'emboîtement de la cale interne proximale et du butoir externe proximal ; et
l'alignement d'ailettes de stabilisation d'un moyeu d'assise de la cale interne distale avec des rayons d'un périmètre à rayons du butoir externe distal au préalable pendant l'emboîtement de la cale interne distale et du butoir externe distal ; et comprenant facultativement en outre l'établissement, le long du butoir de rétention en plusieurs parties proximal, d'évidements d'écoulement (733, 933) pour fournir une voie d'écoulement longitudinale pour le fluide de gonflage et limiter l'écoulement radial de fluide de gonflage.

15. Procédé d'assemblage du cathéter à ballonnet selon la revendication 10, comprenant en outre :
l'alignement des bras d'extension d'un corps conique effilé strié (541, 741) de la cale interne proximale avec les rayons (531, 731) d'un périmètre à rayons (530, 730) du butoir externe proximal au préalable pendant l'emboîtement de la cale interne proximale et du butoir externe proximal ; et
l'alignement des bras d'extension d'un corps effilé strié de la cale interne distale avec des rayons d'un périmètre à rayons du butoir externe distal au préalable pendant l'emboîtement de la cale interne distale et du butoir externe distal.
